# EUROPEAN PATENT SPECIFICATION

(11) **EP 1 472 365 B1**
(45) Date of publication and mention of the grant of the patent: **05.11.2008**
(21) Application number: 02807375.7
(22) Date of filing: 01.11.2002
(51) Int. Cl.: C12Q 1/68, C12P 19/34, C07H 21/04

(54) **RECEPTOR CAPTURE ASSAY**
REZEPTOREINFANG-ASSAY
DOSAGE RELATIF A LA CAPTURE D'UN RECEPTEUR

(30) Priority: 02.11.2001 US 336065 P; 03.04.2002 US 369789 P
(43) Date of publication of application: 03.11.2004
(73) Proprietor: Hybrizyme Corporation, Raleigh, NC 27706 (US)
(72) Inventor: ALLEN, Randy, Leiman, Apex, NC 27502 (US); WILLEY, John, Jeffrey, Raleigh, NC 27612 (US)
(74) Representative: Winckels, Johannes Hubertus F.
(86) International application number: PCT/US2002/035262
(87) International publication number: WO 2004/001371

(56) References cited:
- EP-A- 1 138 781
- WO-A-99/13338
- US-A- 6 127 136
- US-A- 6 140 063
- US-B1- 6 432 692
- LISLE C M ET AL: "SHORT TECHNICAL REPORTS NOVEL SIGNAL AMPLIFICATION TECHNOLOGY WITH APPLICATIONS IN DNA AND PROTEIN DETECTION SYSTEMS" BIOTECHNIQUES, EATON PUBLISHING, NATICK, US, vol. 30, no. 6, June 2001 (2001-06), pages 1268-1272, XP001036327 ISSN: 0736-6205
- BEHNISCH P.A. ET AL: 'Bioanalytical screening methods for dioxins and dioxin-like compounds - a review of bioassay/biomarker technology' ENVIRONMENT INT'L vol. 27, 2001, pages 413 - 439, XP002979004

## Description

### FIELD OF THE INVENTION

The present invention relates to rapid and sensitive methods for detecting analytes and pathogenic biomarkers.

### BACKGROUND OF THE INVENTION

It is a well accepted tenet of toxicology that the pathology of similarly classified toxic agents is typically effected through related biochemical pathways. As such, there have been numerous attempts to develop bioassays that capitalize upon a mechanistic means to detect toxic agents and identify biomarkers indicating a variety of diseases states and disorders as well as exposure to toxic agents accidentally or intentionally introduced into the environment

After a century or more of rapid growth in the chemical industry throughout the world, a wide variety of industrial toxicants have been manufactured and released into the environment. For instance, the compounds of the polychlorinated hydrocarbon group, and analogous types of halogenated organic compounds are toxic pollutants that are harmful to humans. They are also environmentally persistent and accumulate in the food chain. These include dioxins, furans, polychlorinated biphenyls ("PCBs"), DDT, and analogs and derivatives of these compounds. These types of toxicants are known to cause a host of medical problems in humans and animals, including cancer, delay of developmental milestones, immune suppression, and various ectodermal dysplasias. For example, it has been reported that the general population is chronically exposed to dioxin-like compounds through ingestion of low levels in the food supply.

Dioxin-like compounds and PCBs present a significant industrial and environmental hazard, in that these compounds are resistant to breakdown and accumulate in fats and oils in plants and animals, including humans. Recent studies have reported the presence of dioxins and PCBs in food, Alcock et al., Chemosphere, 1998 37:1457-1472 and animal feed, Bernard et al., Nature 1999 401:231-232. Exposure to dioxins and PCBs has been linked to a wide variety of toxicological and biological effects in animals and humans. These include immunosuppression, induction of undesirable enzyme activity, tumor promotion, hepatotoxicity, and reproductive and developmental toxicity. Several accidental food poisonings with PCBs and dioxins have occurred, the most recent being in Belgium in 1999, when 1 g of dioxins and 50 kg of PCBs were introduced into the food chain.

A critical requirement for dealing with environmental contaminants is effective methods for detecting and quantifying the presence of such toxio compounds. Currently available techniques are generally expensive and time-consuming. For example, the traditional analytical method is high-resolution gas chromatography and mass spectrometry (GC/MS) with a cost of $1200-2000 ffor serum and $800-1500 for soil, depending on how quickly the result is desired and the complexity of the analysis. The concentration of each individual dioxin congener must be detected by GC/MS, then converted to TEQ by a mathematical formula relying upon the assigned TEF values (see Clement, Analytical Chemistry 1991 63: 1130A-1139A.) These high costs are prohibitive to routine monitoring of human, food, and environmental samples.

Many efforts to provide rapid and economical methods of analysis for dioxins, PCBs, DDT and the myriad of other environmental toxicants of this type have relied upon selective binding by specific antibodies. One type of antibody assay is based on selective binding of the toxicant of interest by an antibody or antibodies. For example, U.S. Pat No. 4,238,472 to Albro et al., shows a radio-immunoassay for dioxins (including TCDD) and U.S. Pat. Nos. 5,429,925 and 5,674,697 show an ELISA for dioxins. All immunoassays rely upon a high antigenic specificity for the target molecule to reduce qualitative and quantitative errors. A limitation of this technology is the difficulty in constructing antibodies that recognize a few congeners out of hundreds and distinguish relative toxicity of the congeners that are recognized.

Toxicity of the various chemically and structurally related dioxin-like compounds has been described relative to the ability of the compound to bind to the intracellular aryl-hydrocarbon ("Ah") receptor, or AhR. While the ability of a compound to be a ligand of the Ah receptor is one requirement for dioxin-like toxicity, the toxicity of these compounds also depends on the ability of the compound to promote transformation of the receptor into a DNA-binding form subsequent to ligand binding. The transformation of the Ah receptor comprises a series of events that include dissociation of the inactive receptor from a complex of proteins that include one or more molecules of the chaperonin HSP90, the formation of a new complex that includes HSP90-dissociated Ah receptor plus bound dioxin and the nuclear protein aryl hydrocarbon receptor nuclear translocator ("ARNT" also known as hypoxia-inducible factor-1 Beta), and the binding of the Ah receptor/ARNT complex to specific DNA sequences. For dioxin-type compounds, these specific DNA sequences or molecules are Dioxin-Response Elements ("DREs") and lie upstream of the promoter regions of certain genes, the most studied being the P4501AI gene. The binding of the transformed Ah receptor and associated protein(s) to the DREs enhance transcription of the associated genes.

Another application for rapid and sensitive assay detection methods is tied to the threat of terrorist actions against the United States and its allies using chemical or infectious agents. Acts of terrorism are unpredictable and counter measures must incorporate development of new methods for the rapid detection, accurate diagnosis, and speedy treatment of exposed populations (Claudio, Environmental Health Perspectives 2001 109: A529-A536).

The difficulties in providing sensitive and rapid detection methods are compounded by the danger that chemical warfare agents as well as biowarfare organisms and the resulting toxins produced thereby could be modified to elude detection. Such modifications could allow toxic or infectious agents to elude detection by conventional, structure-based technologies, e.g., antibody or DNA-based tests. In addition, the infective activity of biowarefare organisms could potentially be greatly enhanced, making them toxic at concentrations undetectable utilizing currently available detection systems. In any event, present methods for the identification of toxins or toxicants require laborious sample preparation and sophisticated equipment, resulting in potentially disastrous delays in responding to a threat, or inappropriate treatment based on inadequate information. Moreover, the problems identified above highlight the desirability for the development of novel assays incorporating detection modes that focus on common pathogenic pathways of activation for such toxic agents. While chemical and biological structures may be manipulated, it is often the case that the pathways by which these agents derive their pathogenicity cannot be altered. Monitoring biomarkers that demarcate the destructive functionality of these toxic agents capitalizes on the very biochemical basis that renders them harmful at the cellular level.

There are many toxic agents that are a threat to humans in situations of biological and chemical terrorism. For example, anthrax (*Bacillus anthracis*) is a highly toxic agent and can form spores that are extremely hardy and that can remain viable for a very long time. After inhalation of anthrax spores the disease can rapidly progress, leading to death in a matter of days without timely treatment. Thus, early detection and differential diagnosis is critical. However, to date, there have been no reliable screening tests that are available to diagnose inhalation anthrax during the early stages of that disease (Lane et al., Nature Medicine 2001 7:1271-1273).

In particular, distinguishing other illnesses that present with influenza-like symptoms and signs from inhalation anthrax is difficult. Millions of influenza or influenza-like cases are reported each year. The clinical usefulness of rapid influenza tests for the diagnosis of influenza in individual patients is limited because the sensitivity of the influenza rapid tests is relatively low, and a large proportion of persons with influenza may, therefore, go undetected (Lane and Fauci, JAMA 2001 286:2595-2597).

Yet another application for rapid and sensitive assay detection methods concern a group of compounds known as endocrine disrupters. These compounds have been a concern for some time due to their harmful effects on humans and wildlife. Endocrine disrupters are environmental chemicals thought to mimic natural hormones, thereby inhibiting the action of hormones or altering the normal regulatory function of the immune, nervous, and endocrine systems. Investigators have been expressing concerns over the estrogenic effects of environmental chemicals for more than 25 years.

Much of the recent scientific concern is being generated from increasingly frequent reports showing that diseases and dysfunction that occur at hormonally sensitive'receptor sites common to a variety'of disorders, such as in the breast, uterus and testes. It is now known that extremely low'doses of estrogens (up to 50 times lower than levels considered safe by the American Cancer Society) can cause significant hormonal change. The effects now thought to be associated with environmental endocrine disrupters include breast cancer and endometriosis in women, testicular and prostate cancers in men, abnormal sexual development in children, reduced male fertility, alteration in pituitary and thyroid gland functions, immune suppression, and neurobehavioral effects (Colbom et al., 1993 Environ Health Perspect 101:378-384). Recent reports have also shown that many chemicals such as DDT, dioxins and pesticides that have been released into the environment can disrupt normal endocrine function in a variety of aquatic life and wildlife. These effects include abnormal thyroid function and development in fish and birds, decreased fertility in shellfish, fish, birds, reptiles and mammals, and alteration of immune and behavioral function in birds and mammals.

An environmental endocrine or hormone disrupter may be defined as an exogenous agent that interferes with the synthesis, secretion, transport, binding, action, or elimination of natural hormones in the body that are responsible for the maintenance of homeostasis, reproduction, development, and/or behavior. It is important to note that endocrine disrupters encompass more than environmental estrogens. Endocrine-mimicking compounds can target a variety of receptors and include chemicals that can mimic sex steroids, adrenal steroids, thyroid hormones, vitamin D and retinoic acid. Environmental endocrine disrupters can also possess the ability to affect hormones by altering their synthesis, storage and/or release, transport and clearance, receptor precognition and binding, and post receptor activation. In addition, dose, body burden, timing, and duration of exposure at critical periods of life are also important considerations for assessing the adverse effects of a potential endocrine disrupter.

Hormones elicit responses from their respective target tissues through direct interactions with either intracellular receptors or membrane-bound receptors. Intracellular receptors such as those for sex steroids, adrenal steroids, thyroid hormones, vitamin D and retinoic acid regulate gene transcription in a ligand-dependent manner through their interaction with specific DNA sequences. It is critical that the natural ligands interact with the receptor.

A number of environmental agents have been shown'to alter this interaction by mimicking the natural ligand and acting as either an agonist or antagonist. Compounds such as methoxychlor, chlordecone, DDT, PCBs and alklphenols were shown to interfere with estrogen receptor binding (White et al., 1994 Endocrinology 135:175-182). The antiandrogenic action of the dicarboximide fungicide vinclozolin is the result of the compound's affinity for the androgen receptor (Kelce et al., 1994 Nature 375:581-585). The environmental chemicals consisting of isomers of hexachlorocyclohexane, congeners of dichlorodiphenyl-trichlororoethane (DDT; p,p-DDT; p,p-DDE; o,p-DDT), dieldrin, atrazine and pentachlorophenol caused a statistically significant inhibition of specific binding of DHT to the androgen receptor that ranged from 100 % to 25 %. Many chemicals already classified as environmental estrogens bind multiple receptors with almost equal affinity. For example o,p-DDT and chlordecone inhibit both the estrogen and progesterone receptor with almost identical affinities (Laws et al., 1994 Toxicology 92:127-142). Other compounds such as nonylphenol and HPTE have the ability to inhibit binding to the estrogen, progesterone and androgen receptors with similar affinities (Laws et al., 1995 Toxicologist 15:294).

The aforementioned problems, i.e., detecting industrial contaminants in the environment and food chain, detecting endocrine compounds and endocrine disrupters, and detecting chemical or biological warfare agents have common features and technical requirements, including the need for a high degree of sensitivity and accuracy, relatively low cost and rapid implementation under field conditions. Equally important are the similarities in the pathogenic pathways that many of these toxic agents share with one another that, in turn, provide new opportunities for the development of biomarker-based assays.

Interestingly, many of the effects of these chemical and biological agents have a common mechanism of action in that they are mediated by the same or similar transcription factors, an example of which is the PAS family of proteins. "PAS" is the name of a group of proteins called Per, ARNT, and Sim (Gu, Y.Z., et al, 2000 Annu Rev Pharmacol Toxicol, 40:519-61). The PAS domain is found in a variety of proteins that play roles in development and adaptation to the environment (Jain et. al., Mechanisms of Development 1998 73: 117-123). The PAS domain is also commonly found in proteins that that harbor basic-helix-loop-helix (bHLH-PAS) domains, and that act in pairs as heterodimeric transcription factors. PAS proteins can be classified as either α-class proteins, which often act as the sensors of environmental signals (i.e., AhR, HIF-1α, CLOCK, and SIM), or as P-class proteins (i.e., MOP3 and ARNT) which typically act as the broad-spectrum partners that accompany these heterodimers to their genomic targets (Gu et. al., Annu. Rev. Pharmaco. Toxicol. 2000 40:519-561). Hypoxia inducible factor-1α "HIF-1α" is a transcription factor that is a member of the PAS family of proteins, as further described by U.S. Patent 6,222,018, incorporated herein in its entirety by reference thereto.

The steroid and thyroid superfamily of receptors including the glucocorticoid, estrogen, mineralocorticoid, progesterone, androgen, vitamin D, thyroid, and retinoic acid, and ecdysteroid receptors, and viral erbA oncogene'are inactive in the absence of ligands. Upon binding an agonist ligand, the receptors are activated by a process involving dimerization and a change of conformation analogous to the PAS family of transcription factors. Similarly, the transformed receptor is capable of binding DNA for trans-activation of associated genes as described in Englebienne, Immune and Receptor Assays in Theory and Practice, CRC Press, New York, 2000.

There have been a number of attempts to exploit the aforementioned observations to create novel assays. For example, E1-Fouly et al., 1994 (Environmental Toxicology and Chemistry, 13(10):581-1588) describe a bioassay for dioxins conducted by splicing AhR recognition sites to a human PAP vector, and transfecting the vector into mouse hepatoma cells. However, a PAP enzyme-based reporter gene is considered to be unreliable, based on the report that many cells, and especially human epithelial cells, contain endogenous levels of heat-stable PAP activity that can interfere with the bioassay analysis.

Denison, et al. U. S. Pat. No. 5,854,010, describe a bioassay system prepared by inserting dioxin responsive elements in front of a luciferase reporter gene, and then, transfecting the resultant recombinant expression plasmid into mouse hepatoma cells. Bradfield, et al., U.S. Pat. No. 5,378,822 describes the use of expressed proteins from cDNA for mouse or human AhR in a bioassay to detect dioxins in samples. Bradfield, et al., U.S. Pat No. 5,650,283 describe transgenic host cells, including transfected mammalian or yeast cells expressing AhR protein that have a DRE-driven lac Z reporter for detecting halogenated hydrocarbon compounds. All bioassays require maintenance of a cell culture that is exposed to the toxicants of interest.

Poland, et al., U.S. Pat. No. 5,128,244, describes a competitive binding assay where dioxin is presented to AhR prepared from mouse cytosol and an ¹²⁵ I-dioxin derivative is allowed to compete with the dioxin in the sample for a limited number of AhR binding sites. The unbound radiolabeled dioxin analog is separated from the bound radioactivity before counting. The short half-life of I¹²⁵ and the dangers associated with exposure and disposal of radioactive materials limits the usefulness of this assay for routine testing.

Wheelock and Babish, U.S. Pat. No. 5,529,899 and Wheelock, U.S. Patent No. 6,127,136 describe assays that detect formation of an AhR/ARNT complex, with an antibody specific for the bound or complexed form of the Ahr or ARNT protein, respectively. The difficulty in generating specific and high affinity antibodies to the AhR has limited the usefulness of this assay for testing low levels of dioxins. Similarly, the use of antibodies specific for ARNT to detect the AhR/ARNT complex requires the use of relatively large volumes of AhR cytosol.

U.S. Patent 6,316,197, incorporated by reference herein, describes methods for determining early exposure to infectious agents by monitoring patterns of gene expression, e.g., monitoring the up-regulation and expression of a number of genes, including the HIF-α gene. The '197 patent indicates that HIF-1α mRNA is up-regulated in circulating blood lymphocytes within a few hours upon exposure to anthrax spores. This observation is made more significant since the principle mechanism for HIF-1α regulation is primarily at the protein level, as opposed to regulation at the mRNA level (Zelzer et al., EMBO J. 1998 17:5085-5094). However, the methods of the '197 patent require obtaining a sample from a mammal then, "detecting a pattern of gene expression or protein expression present in said sample; comparing the pattern of gene or protein expression from said sample with a library of known patterns of gene or protein expressions for toxic agents." However, monitoring broad patterns of gene expression fails to solve the problem of providing rapid, sensitive and economical assays for early detection of specific infectious agents.

Gel electrophoresis mobility shift assays (EMSA) are widely used for the detection of protein-nucleic interactions (Revzin, BioTechniques, 1987 7:346-355). In the performance of EMSA, P³² labeled DNA and protein are mixed together, the solution subjected to electrophoresis through polyacrylamide, and the gel is then analyzed for DNA, usually by autoradiography. Binding of the protein to the DNA can result in a complex that has a different electrophoretic mobility from the free DNA. EMSA has been employed to detect the induction of HIF-1α requiring days to complete with limited throughput (Zelzer et al., (1998) *supra)*. EMSA is too expensive and time-consuming for routine use.

Nargessi, U.S. Patent No. 5,770,176 describes immunoassays for detecting and quantitating functional nuclear receptors in cell or tissue samples by correlating the specific binding of DNA response element(s) to receptors, with binding to a specific antibody. These assays are designed for screening for nuclear receptors in samples, e.g., so that such receptors can be identified in tumor biopsy samples.

Thus, there remains a long-standing need in the art for assays that can rapidly, reliably, and economically detect and quantify environmental toxicants such as the persistent halogenated hydrocarbons, infectious agents, and toxins as well as the need for assays able to measure the activity of certain biomarkers and to demonstrate exposure of an organism to any number of such toxic agents.

### SUMMARY OF THE INVENTION

The present invention addresses these and other shortcomings in the art by providing novel methods for rapid, sensitive, consistent and economical methods for measuring analytes and transcription factor activity based on amplification of a nucleic acid-tracer and the subsequent measurement of amplified product that is ultimately correlated with the presence of analyte or transcription factor activity.

One embodiment of the present invention is practiced by contacting a transcription factor with an analyte of interest, under conditions favorable to allow the activated transcription factor to become capable of binding a DNA response element embodied in nucleic-acid tracer reagent primer recognition sequences preselected for standard nucleic acid amplification protocols. More particularly, the method calls for contacting a sample having an analyte disposed therein with a transcription factor assay reagent capable of specific binding to the analyte. The transcription factor is transformed by contact with the analyte to yield an activated transcription factor that is functional to bind a compatible DNA response element. A nucleic acid tracer reagent comprising at least one nucleic acid primer recognition sequence and at least one DNA response element to which the activated transcription factor binds is also contacted with the transcription factor assay reagent and the sample simultaneously or sequentially, under conditions promoting formation of a protein-nucleic acid complex between the DNA in the nucleic acid-tracer reagent and activated transcription factor. The protein-nucleic acid complex is then separated from uncomplexed nucleic acid tracer reagent and detection of the complexed tracer is performed by nucleic acid amplification wherein the presence of amplified nucleic acid tracer reagent indicates the presence of analyte in the sample.

In another embodiment, the invention also provides rapid, sensitive, and economical methods and assays for detecting changes in transcription factor activation in an organism exposed to disorders, toxicants, toxins, or infectious agents. The assay has unique utility in detecting changes in transcription activation in an animal or human in order to determine if transcription factors isolated from an organism have already been activated by, for example, exposure to certain disorders, toxicants, toxins, and infectious diseases.

In this embodiment of the method of the present invention, the assay is employed to detect or measure transcription factor activation, especially in tissue believed to have been exposed to a toxic agent capable of activating the transcription factor. The method broadly includes contacting the sample with a nucleic acid tracer reagent comprising at least one nucleic acid primer recognition sequence and at least one DNA response element to which the activated form of the transcription factor binds. The contact is performed under conditions promoting formation of a protein-nucleic acid complex between tracer reagent and the transcription factor. This complex is separated from uncomplexed nucleic acid tracer reagent and is detected by nucleic acid amplification with a subsequent correlation of the presence of amplified nucleic acid tracer reagent with the activity of said transcription factor.

The present invention also provides for a novel nucleic acid-tracer reagent for deployment in the various assay methods of the invention.

### DETAILED DESCRIPTION OF THE INVENTION

The present invention provides rapid, sensitive, consistent and economical methods and assays for measuring analytes and transcription factor activity based on amplification of a nucleic acid-tracer and the subsequent measurement of amplified product that is ultimately correlated with the presence of analyte or transcription factor activity. One embodiment of the present invention is conducted by contacting a transcription factor with an analyte of interest, under conditions favorable to allow the activated receptor to transform into a protein capable of binding a DNA response element embodied in nucleic acid sequences preselected for standard art amplification protocols.

In another embodiment, the invention also provides rapid, sensitive, and economical methods and assays for detecting changes in transcription factor activation in an organism exposed to disorders, toxicants, toxins, or infectious agents. Such assays have utility in detecting changes in transcription activation in an animal or human for any desirable purpose. Detection of these activation events provides for diagnostic capabilities and, due to the same, these assays may be employed to determine if transcription factors isolated from an organism have already been activated by, for example, exposure to dioxins or anthrax.

Thus, the present invention provides improved assay methods for detection of analytes in a sample and for diagnosing exposure to certain disorders, toxicants, toxins, and infectious diseases.

When the detection or measurement of analyte takes place by the method of the present invention, an appropriate transcription factor is contacted in the presence of required reagents or cell factors, with known concentrations of an analyte or analytes of interest, or alternatively, is contacted with an unknown sample that might possibly contain a suspected analyte of interest. The resulting set of analytes is then detected by any of several methods of the invention, in order to correlate concentrations of known and unknown samples of the analyte and to determine the presence, of the analyte in a sample (e.g., of soil, food, blood, tissue, etc.).

When the method of the present invention is employed to detect or measure transcription factor activation, tissue believed to contain the transcription factor is treated to obtain the transcription factor in a form sufficiently purified to allow for determination of the activated state by the methods of the invention. This determination may be readily employed to detect disorders or the exposure of an organism (e.g., plant life, wild animals, pets, domestic livestock, and/or humans) to toxicants, toxins, or infectious agents of interest.

A high degree of specificity is required when a transcription factor is activated, and is then able to selectively bind to a specific and unique nucleic acid-tracer reagent comprising a DNA response element and primer recognition sequence. Once bound by the transcription factor in this selective manner, the DNA response element is, in turn, readily detected with great sensitivity and accuracy by DNA amplification methods, e.g., PCR.

As mentioned beforehand, another embodiment of the present invention may be employed to detect transcription factor activation occurring in organisms of interest. In this embodiment of the invention, the requirement for direct isolation and detection of a toxicant, toxic agent or infectious agent is avoided by detecting specific effects on transcription factor activation states.

A transcription factor that has contacted an analyte of interest and that is transformed into an activated DNA-binding protein, will bind with a compatible nucleic acid-tracer reagent under conditions promoting formation of a protein-nucleic acid (DNA) complex. The protein-nucleic acid complex is separated from unbound DNA and unbound protein by any of several standard methods well known in the art, including by way of example, gel filtration chromatography, centrifugation filtration, hydrophobic separation, charged membrane-based separation, hydroxyapatite separation, antibody precipitation, salt precipitation, organic solvent precipitation, polyethylene glycol precipitation, and silica precipitation.

Preferably, separation of the protein-nucleic acid complex from uncomplexed DNA is accomplished by any standard art method of capturing the complex onto a solid substrate (examples of which include test tubes, microtiter wells, microtiter strips, microtiter plates, beads, microparicles, magnetic particles and combinations thereof) followed by one or more washing steps to remove unbound materials. Thereafter, detection and/or quantification of the DNA of the complex is conducted by PCR, and/or any compatible nucleic acid amplification. Moreover, subsequent correlation with a control or standard curve provides a highly accurate determination of the analyte concentration.

As such, the methods of the present invention, may be employed to screen compounds (e.g., industrial and naturally occurring compounds, medications, and the like) for previously unknown desirable or undesirable effects on certain receptor or transcription factor systems. For instance, compounds may be screened to determine whether they share the activity of dioxins and PCBs by inappropriately activating the Ah receptor. Insecticides may be screened to determine if they share the activity of, for example, DDT by inappropriately activating avian estrogen receptors. Similarly, the inventive methods are readily employed to screen or detect activated transcription factors for the diagnosis, prognosis and treatment of disease, and as a platform for the discovery of new pharmacological drugs and strategies.

In order that the artisan can more readily appreciate the scope of the invention, the following definitions are provided.

The term "transcription factor" herein refers to proteins that interact with response elements, i.e., DNA response elements, associated with regulated genes, and that are or become DNA-binding proteins under specific circumstances. "Receptors" as employed herein are defined as ligand-activated transcription factors and may exist in the cytoplasm (e.g. the Ah receptor) or the nucleus (e.g. the glucocorticoid receptor). Nuclear receptors broadly include receptors for steroid hormones, thyroid hormones, retinoids, hormonal forms of vitamin A and D, peroxisomal activators, and ecdysone. Many transcription factors are activated to bind DNA by biochemical pathways that are not ligand-mediated (e.g. HIF-1α).

As used herein the term "activated" used in conjunction with the term receptor or transcription factor means that protein that is transformed into a form that is capable of binding to its respective DNA response element. Although the activating response may differ, there are striking similarities between signaling pathways of the various transcription factors. For example, the steroid and thyroid hormone receptor superfamily and the PAS α-class proteins AhR, HIF-1α, and CLOCK bind β-class PAS proteins and the resulting homodimers or heterodimers recognize their respective DNA response elements.

"DNA response elements" are defined herein to mean a unique DNA sequence'to which a transcription factor. Under physiological conditions, DNA response elements are DNA sequences associated with or "compatible" with a gene or region of a gene to which a transcription factor binds and regulates transcription of the gene. DNA response elements are typically 5' to the promoters of regulated genes, but may also be downstream. Activating agents directly or indirectly activate transcription factors to bind specific DNA response elements normally present in the nucleus of the respective cell type, and by this method will up-regulate or down-regulate transcription of corresponding gene(s) and any protein synthesis subsequent thereto.

"Nucleic acid-tracer reagent" is defined as a nucleic acid comprising one or more DNA response elements to which primer recognition sequences are attached.

For convenience of description, several additional terms are defined as follows. "Analytes" are broadly defined as the substance being identified and measured in an assay. In certain embodiments of the invention, analytes are contemplated to be persistent environmental toxicants extracted from or present in a wide variety of materials, including soil, rock, and man made artifacts and building materials that may have become contaminated by way of the raw materials from which they are fashioned or by the processes through which they are produced (e.g., furnishings, interior and exterior features of buildings or other structures, vehicles, clothing and the like) as well as those toxicants thought to be present on or in any type of biological materials.

The term, "biological material" as used herein broadly refers to substances or materials, including foodstuffs, obtained from or as part of a living or formerly living organism, as well as medicinal and grooming products, for humans and animals, whether artificial, animal or plant in nature.

The artisan well understands and appreciates that when biological materials are tested for medical or diagnostic purposes, sample sources may include tissue or blood obtained from living animals or even human patients in need of such testing.

The term "dioxin-like" broadly refers to dibenzodioxins, dibenzofurans, azobenzenes, dibenzo-ethers, certain polychlorinated biphenyls, certain polyaromatic hydrocarbons, and many toxic and persistent derivatives thereof, as set forth in greater detail hereinbelow.

The term "dioxin" broadly refers to 2,3,7,8-tetrachlorodibenzo-p-dioxin or TCDD and/or any of the 75 congeners of TCDD.

The term "furan" refers to 2,3,7,8-tetrachlorodibenzo-p-furan (TCDF) and/or any of the 135 congeners of TCDF.

The phrase, "PCB compounds" broadly refers to any of the specific PCB congeners and potential degradation products of the same that may be present in PCB contaminated materials. These include any of the 209 different isomeric forms of PCB found in the commercial Aroclor^{™} compositions, as well as others that may have resulted from non-commercial production, or the metabolic actions of living organisms, e.g., biotransformation by soil microorganisms.

Further, the use of singular terms for convenience in this description is in no way intended to be so limiting. Thus, for example, reference to a composition comprising "an antibody" includes reference to one or more of such antibodies (e.g., to a preparation with sufficient antibodies for the intended purpose) unless otherwise stated. Recombinant constructs were utilized in the Examples provided hereinbelow. The constructs comprise a vector, such as a plasmid or viral vector, into which a sequence for expression of a receptor, receptor binding partner, or protein fusion of either dimeric member was inserted. Large numbers of suitable vectors and promoters are known to those of skill in the art, and are commercially available.

Host cells containing the recombinant construct can be a higher eukaryotic cell, such as a mammalian cell, or a lower eukaryotic cell, such as a yeast cell. Alternatively, the host cell can be a prokaryotic cell, such as a bacterial cell. Introduction of the construct into the host cell can be effected by calcium phosphate transfection, lipid or DEAE-Dextran mediated transfection, or electroporation.

Transcription factors and binding partners can be expressed in mammalian cells, yeast, bacteria, or other cells under the control of appropriate promoters. Alternatively, a baculovirus/insect cell expression system can also be employed. Cell-free translation systems can also be employed to produce such proteins using RNAs derived from the cDNA. Receptors produced by the above stated methods may be commercially available. Appropriate cloning and expression vectors for use with prokaryotic and eukaryotic hosts are described by Sambrook et al., Molecular Cloning--A Laboratory Manual (2nd ed.) Vol. 1-3, 1989, incorporated herein in its entirety by reference thereto.

### I. TRANSCRIPTION FACTORS

Transcription factors that function as first messengers may be directly activated by a ligand. Several directly activated receptor families function through a common mechanism of action. The receptor protein is inactive and usually complexed with chaperone proteins. When a ligand is introduced, the receptor binds ligand, undergoes a transformation, and becomes activated. Furthermore, the receptor may then bind either another receptor of the same type to form a homodimer or a different protein/receptor that usually shares homology and is a member of the same class of proteins to form a heterodimer. The activated receptor may then bind a specific DNA target to affect transcription.

Simply by way of example, steroid, retinoid, and thyroid hormones regulate the development and homeostasis in eukaryotes. However, when the primary structure of these receptors were elucidated by cloning and sequencing, it was rapidly recognized that they share a high level of homology. Furthermore, all have been identified as regulatory proteins acting on gene transcription upon ligand-binding. These common features support their classification under a large superfamily of receptor proteins. This superfamily includes the glucocorticoid receptor, estrogen receptor, the mineralocorticoid receptor, the progesterone receptor, the androgen receptor, the vitamin D receptor, the thyroid hormone receptor, the retinoic acid receptor of mammals, and also the ecdysteroid receptor of insects. This commonality has further been extended to the viral erbA oncogene whose protooncogene product has been identified as a thyroid hormone receptor antagonist. Several orphan receptors (i.e., receptors whose specific ligand has not been identified) have also been classified under this superfamily after sequencing.

The intracellular receptors of this superfamily are inactive in the absence of ligands. Generally, upon binding an agonist ligand, the receptors are activated by a process involving dimerization and a change of conformation. In steroid hormone receptors, the dimerization process is accompanied by the loss of the heat-shock proteins that are associated with the monomeric form of the receptor. This is termed transformation and is required for binding to DNA and for transactivation. Generally, when the ligand is an antagonist, dimerization occurs, but the receptor is unable to undergo transactivation. The thyroid hormone, retinoic acid and vitamin D receptors are nonetheless able to dimerize and bind DNA in the absence of ligand. In this case, ligand binding occurs while the receptor is DNA-bound in the cell nucleus. Like the steroid receptors, they undergo also transformation upon ligand binding, Englebienne, Immune and Receptor Assays in Theory and Practice, CRC Press, New York, 2000.

Furthermore, transcription factors that act as first messengers include proteins that are members of the PAS family of proteins, named for Per, ARNT, and Sim proteins a taught by Gu, Y.Z., et al, 2000 Annu Rev Pharmacol Toxicol, 40:519-61), incorporated herein by reference thereto. The PAS domain is found in a variety of proteins that play roles in development and adaptation to the environment. The PAS domain is also commonly found in proteins that that harbor basic-helix-loop-helix (bHLH-PAS) domains, and that act in pairs as heterodimeric transcription factors. The ability of a transcription factor to dimerize with multiple partners is present across many families of proteins. Some basic helix-loop-helix-PAS family member proteins can interact with multiple partners, forming homodimers in vitro or heterodimers *in vivo*. Upon binding a suitable ligand, activated AhR binds to ARNT, the aryl hydrocarbon receptor nuclear translocator. The AhR/ARNT complex forms a heteromer that is then able to bind to a specific DNA response element called the dioxin-response element.

Recently, a number of "orphan" bHLH-PAS proteins (MOP3, MOP4, and MOP5) have' emerged from searches of expressed sequence tag databases and low stringency hybridization screens. Identification of their heterodimeric partners and determination the DNA response element is only now being determined (Hogenesch et. al., Proc. Natl. Acad. Sci. USA 1998 95: 5474-5479)

Certain members of the PAS family of transcription factors are activated indirectly. HIF-1α operates as follows. Under normal conditions, cellular HIF-1α is rapidly degraded through the ubiquitin-proteasome pathway. When the cell is exposed to an activator, the HIF-1α protein is stabilized. HIF-1α then translocates from the cytoplasm to the nucleus, where it complexes with ARNT to create the HIF-1 complex. The HIF-1 complex then binds to its specific DNA response element, known as the hypoxia responsive element ("HRE"). CLOCK protein is another receptor or transcription factor that operates analogously to HIF-1α. In response to light, CLOCK protein is induced and forms a complex with MOP3. The heterodimer translocates to the nucleus and binds to its specific DNA response element called MOP3 and MOP4 responsive element ("M34RE").

Other transcription factors are also indirectly activated by phosphorylation of certain residues, or by modification of inhibitory proteins bound to the factors. In many cases, the external agent interacts with membrane bound receptors in such a manner to stimulate one or a cascade of modification enzymes, typically kinases. Simply by way of example, transcription factors such as Stat members, AP-1, and CREB are activated through phosphorylation by kinases that are, in turn, activated by growth factors, cytokines, or other stimuli. NFkB family members are bound with IkB members in the cytoplasm and are transcriptionally inactive. Degradation of IkB occurs when cells are treated with various agents or cytokines. NFkB dimers can translocate to the nucleus and bind specific DNA elements of regulated genes.

As such, the methods of the present invention can optionally be practiced with any first or second messenger, e.g., transcription factors, that when activated will bind to specific DNA elements suitable for use in the inventive assay as a nucleic acid reporter molecule.

In one embodiment of the present invention, the PAS ligand-activated transcription factor is an aryl hydrocarbon receptor. The PAS domains of the AhR function in association with 90 kDa heat shock protein (hsp90), dimerization with ARNT, and binding ligand.

In another embodiment, the hormone-dependent nuclear receptors according to the invention are the estrogen receptors ("ERs") that bind to their respective estrogen specific DNA response elements and the glucocorticoid receptor ("GR") that binds directly to glucocorticoid response elements (GREs) to regulate gene transcription.

In yet a more particular embodiment of the invention, the method utilizes HIF1-α, a PAS protein transcription factor activated through a second messenger.

### A. Aryl Hydrocarbon Preceptor

The aryl hydrocarbon receptor is a ligand activated transcription factor that is a member of the basic helix-loop-helix-PAS family of proteins. The AhR and its binding partner, ARNT, are members of the PAS family of proteins, as discussed supra, and share homology in this domain. The PAS domains of the AhR function in association with 90 kDa heat shock protein (hsp90), dimerization with ARNT, and binding ligand. The AhR and ARNT also have an amino terminal basic region and helix-loop-helix domain which function together and mediate DNA binding and protein dimerization.

The inactive, non-ligand bound AhR is complexed in the cytoplasm with heat shock protein 90 and an immunophilin-like protein referred to in the art by any one of three terms, namely XAP2, AIM, or Ara9. Upon binding ligand, the activated AhR dissociates from the complex and binds ARNT. The transformed AhR/ARNT complex is a heteromer that is then able to bind a DNA response element specific for dioxins, the dioxin-responsive element. It has been speculated that the toxic effects of dioxin-like compounds are likely due to the inappropriate induction or repression of regulated genes and that AhR-binding chemicals may be mimicking a currently unidentified endogenous hormone.

Structurally different compounds bind the AhR with different affinities that strongly correlate to the observed toxicity. Most populations are exposed to mixtures of such compounds present in food or the environment and the identity and concentration of the moieties present in a sample impact the total AhR-mediated response. International convention has defined toxic equivalency factor ("TEF") values for AhR binding chemicals, wherein the value is I for TCDD, the most potent chemical of that group, and a value <1 for all others (see Van den Berg, M., et al., 1998 Environ Health Perspect 106:775-789, incorporated herein in its entirety by reference thereto). The total of toxic equivalents ("TEQ") is the sum of the toxicity for each congener in a mixture (TEF times concentration of congener) and is an accepted industry standard.

The heteromer containing the AhR can be obtained from any number of sources. For example, the heteromer is present in rodent liver, thymus, lung, kidney, brain, testis, and skeletal muscle cells. A particularly preferred source of the heteromer is a cytosol fraction of mammalian hepatocytes. Preferably, the heteromer can be obtained by isolating liver cytosol from male Hartley guinea pigs (see E. C. Henry, et al., "Characterization of Multiple Forms of the Ah receptor: Comparison of Species and Tissues," Biochem, 28:6430-40, 1989, incorporated herein in its entirety by reference thereto) and frozen or lyophilized in five milliliter aliquots contained in glass or plastic test tubes. When AhR is obtained from liver cytosol, generally, all of the proteins and enzymes necessary for transformation of the AhR are present. Hepatocyte cell lines are also a convenient source of the AhR heteromer because these provide a uniform, replicable source of the cytosol preparation that does not require harvesting and disrupting liver tissue. Because the transformation/activation of the heteromer is a natural part of cell processes, the required proteins and enzymes are present in the proper ratios for complete transformation and activation of Ah receptor in the presence of an agent for which AhR responds.

Bradfield et al., U.S. Pat. No. 5,650,283, incorporated herein in its entirety by reference thereto, provides methods for generating large quantities of recombinant AhR. The patent describes full length AhR and AhR containing deletions at its amino or carboxyl ends and chimeric receptors that are functional, all of which bind their associated DNA response element when activated by ligand. These chimeric Ah receptors are prepared by exchanging the DNA binding and primary dimerization domains of the AHR with analogous domains from the LexA and Gal4 proteins. The resulting chimeric proteins yield pharmacology that closely approximates that of the AHR/ARNT/DRE system (Carver et. al., JBC 1994 269(48): 30109-30112).

Binding ligands for AhR include a range of halogenated compounds, such as PCBs, dioxins and dioxin-like compounds known in the art. Dioxin-like compounds include, for example, polychlorinated dibenzodioxins, polychlorinated dibenzofurans, polychlorinated biphenyls, and structural analogues thereof which exhibit biological activity that is characteristic of polychlorinated dibenzodioxins, polychlorinated dibenzofurans, and/or polychlorinated biphenyls.

The term dioxin, as commonly used, is shorthand for 2,3,7,8-tetrachlorodibenzo-p-dioxin. TCDD is only one member or congener of the polychlorinated dibenzo-p-dioxin family ("PCDD"), of which there are 75 possible congeners whose structures vary according to the number and location of the chlorine atoms. Biologically, TCDD is the most potent PCDD while most other PCDDs are less active by a factor ranging from ten to thousands. TCDD has been studied most extensively of all the PCDD congeners.

Several aromatic hydrocarbons share biological properties with TCDD, particularly when substituted with chlorine in the lateral positions. The most important of these are the polychlorinated dibenzofurans ("PCDF") and certain members of the polychlorinated biphenyl family. The large number of possible PCDD, PCDF, and PCB congeners greatly complicates environmental analysis and therefore complex clean-up procedures are required before such analysis can be undertaken. As used herein, "dioxin-like compounds" includes all members of the above-identified families of compounds and other compounds that induce similar cellular effects, such as azobenzenes and benzopyrenes.

### B. Estrogen Receptor

The estrogen receptor ("ER") binds directly to estrogen response elements ("EREs") to regulate gene transcription. The ER is a member of a large family of nuclear receptors and is hormone dependent in function. The ER recognizes many structurally diverse compounds present in the body endogenously, or exogenously as drugs, pollutants, or food components. Estrogens strongly influence the growth, differentiation, and functioning of the reproductive system, specifically the mammary gland and uterus. ER antagonists, such as tamoxifen, have been used successfully in the treatment of ER positive breast cancers.

The ER contains a ligand binding domain, a DNA binding domain, a dimerization interface domain, and N- and C- terminal transactivation domains. Most laboratories observe ER homodimer formation on the ERE in the absence of ligand, and that ligands alter transcription function.

Recently, a cDNA HE0 has been derived from wild-type estrogen receptor containing a single amino acid substitution allowing the receptor to bind its DNA response element only after ligand activation (Aumais et. al., JBC 272(18): 12229-12235).

### C. Glucocorticoid Receptor

Glucocorticoids are steroids involved in regulation of energy metabolism and immune/inflammatory responses. The glucocorticoid receptor ("GR") binds directly to glucocorticoid response elements (GREs) to regulate gene transcription. The GR is a member of a large family of nuclear receptors and is hormone dependent in function. The GR recognizes many structurally diverse compounds present in the body endogenously or exogenously as drugs, pollutants, or food components. The GR contains a ligand binding domain, a DNA binding domain, a dimerization interface domain, and N- and C- terminal transactivation domains and is complexed with hsp90 proteins until ligand induces dissociation and homodimer formation.

### D. HIF-1α Transcription Factor

HIF-1α plays an important role in activating homeostatic responses to hypoxia, the state in which oxygen demand exceeds supply, and the activation of HIF-1α can be exploited as a biomarker for the early detection of infectious disease. It is well understood that mammals require molecular oxygen for essential metabolic processes, including oxidative phosphorylation in which O₂ serves as electron acceptor during ATP formation. Systemic, local, and intracellular homeostatic responses elicited by hypoxia include erythropoiesis by individuals who are anemic or at high altitude (Jelkmann, Physiol. Rev. 1992 72:449-489), neovascularization in ischemic myocardium (White et al., Circ. Res.1992 71:1490-1500), and glycolysis in cells cultured at reduced O₂ tension (Wolfle et al., Eur. J. Biochem. 1983 135:405-412). These adaptive responses either increase O₂ delivery or activate alternate metabolic pathways that do not require O₂. Hypoxia-inducible gene products that participate in these responses include erythropoietin (EPO) (reviewed in Semenza, Hematol. Oncol. Clinics N. Amer. 1994 8:863-884), vascular endothelial growth factor (Shweiki et al., Nature 1992 359:843-845; Banai et al., Cardiovasc. Res. 1994 28:1176-1179; Goldberg & Schneider, J. Biol. Chem. 1994 269:4355-4359), and glycolytic enzymes (Firth et al., Proc. Natl. Acad. Sci. USA 1994 91:6496-6500; Semenza et al., J. Biol. Chem. 1994 269:23757-23763).

The molecular mechanisms that mediate genetic responses to hypoxia have been extensively investigated for the EPO gene, which encodes a growth factor that regulates erythropoiesis (Jelkmann, 1992 supra; Semenza, 1994, *supra*). *Cis*-acting DNA sequences required for transcriptional activation in response to hypoxia were identified in the EPO 3'-flanking region and a *trans*-acting factor that binds to the enhancer, hypoxia-inducible factor 1 alpha (HIF-1α), has been shown to fulfil criteria for a physiological regulator ofEPO transcription: inducers of EPO expression (1% O₂, cobalt chloride [CoCl₂], and desferrioxamine ("DFX") also induced MIF-1 DNA binding activity with similar kinetics; inhibitors of EPO expression (actinomycin D, cycloheximide, and 2-aminopurine) blocked induction of HIF-1α activity; and mutations in the EPO 3'-flanking region that eliminated HIF-1 binding also eliminated enhancer function (Semenza, 1994, supra). These results also support the hypothesis that O₂ tension is sensed by a hemoprotein (Goldberg et al., Science 1988 242:1412-1415) and that a signal transduction pathway requiring ongoing transcription, translation, and protein phosphorylation participates in the induction of HIF-1α DNA-binding activity and EPO transcription in hypoxic cells (Semenza (1994) *supra*). EPO expression is cell type specific, but induction of HIF-1α activity by 1% O₂, CoCl₂ or DFX has been detected in many mammalian cell lines (Wang & Semenza, Proc. Natl. Acad. Sci. USA 1993 90:4304-4308).

In addition, RNA molecules encoding several glycolytic enzymes have been reported to be induced by 1% O₂, CoCl₂, or DFX in EPO-producing Hep3B or non-producing HeLa cells, whereas treatment with cycloheximide blocked the induction of these same RNA molecules. Further, glycolytic gene sequences containing HIF-1α binding sites were shown to mediate hypoxia-inducible transcription in transfection assays (Firth, et al.,1994, supra; Semenza, et al., 1994, supra). These reports support the role of HIF-1α in activating homeostatic responses to hypoxia.

### II. ASSAY METHODS

### A. Assays for Detecting Analyte

In one embodiment of the present invention, the assay method comprises a series of steps that exploit the activation of a specific transcription factor by an analyte, followed by selective binding of the activated transcription factor with a nucleic acid-tracer containing a primer recognition sequence and a corresponding DNA response element to form a complex, and then determining the presence of nucleic acid-tracer that was selectively bound, by any of several amplification methods known in the art. These steps are set forth below.
a) contacting a transcription factor assay reagent and analyte in presence of normal cellular components and under conditions suitable for transcription factor to be activated;
b) allowing activated transcription factor to bind to a nucleic acid-tracer reagent comprising a nucleic acid amplification primer recognition sequence and DNA response element to form a protein-nucleic acid complex;
c) separating protein-nucleic acid complex from uncomplexed nucleic acid-tracer; and
d) detecting and/or quantifying complexed nucleic acid-tracer by amplification.

The transcription factor assay reagent is obtained from cells or tissues appropriate to the transcription factor of interest or produced by recombinant methods. The transcription factor assay reagent includes, e.g., the steroid hormone superfamily receptors, PAS superfamily proteins, Stat family transcription factors, Rel or NFkB family proteins, CREB family proteins, and AP-1 family proteins. For example, AhR receptor is typically obtained by disrupting and isolating cytoplasmic fractions of mammalian liver cells obtained from rodent sources (e.g., mouse, rat, or guinea pig liver, or hepatocyte tumor cell lines), as exemplified hereinbelow.

Analyte is prepared in the form of calibration standards in a range of concentrations representative of compounds that it is desired to detect or quantify. For dioxins, calibrators in concentrations ranging from 10 through about 1000 fmol of 2,3,7,8-tetrachlorodibenzo-p-dioxin ("TCDD") in a compatible solvent, such as, e.g., methanol or DMSO are readily employed.

The artisan will appreciate that TCDD and other dioxins will generally stick to plastic. Thus, in AhR/TCDD assays, contact between the TCDD or other dioxins is preferably conducted in glass. Once the analyte has made sufficient contact with the AhR so that the activated complex is stable, it can be moved to plastic materials (e.g., any suitable polymer-based equipment) such as commercially available microtiter strips.

Standard microtiter strips are convenient because they are available in the single strips of 8 or 12 wells or a standard 96-well format. Similar high-density microtiter plate formats are available for high throughput screening methods.

After the assay medium containing activated transcription factor is transferred to the microtiter strips, the glass tubes can be discarded. Employing microtiter plates allows for the use of all the equipment (e.g. washers, pipetters, etc.) that have been developed for ELISA work. Preferably, the microtiter strips or plates are of a dimension that is suitable for PCR, as exemplified herein. Most thermocyclers are now configured for the 96-well format.

In order to determine the presence of only that nucleic acid-tracer which has participated in binding (i.e. the DNA-protein complex formation) with an activated transcription factor, the complex is separated from the uncomplexed proteins and nucleic acid-tracer in the assay medium. While any standard art separation methods may be employed, as noted *supra*, separation is preferably facilitated by capturing or anchoring the complex to a solid support so that other proteins and nucleic acids can be removed by washing. The capture can be by any method compatible with the assay, but is preferably by means of an antibody selected to be specific for one or more epitopes present in the complex.

The capture antibody can be directly anchored to a solid support by any method known in the art (e.g., Protein A, and/or one or more polymer linkers) or alternatively, the capture antibody can be contacted with the complex and thereafter itself bound to another ligand (e.g., any binding antibody that is, in turn, selective for the capture antibody) wherein the binding antibody is bound to, or can be later bound to, a solid support.

For assays where the receptor is AhR and the analyte(s) are TCDD and related compounds, the preferred capture antibody selectively binds ARNT. An anti-ARNT antibody can be elicited by standard immunization methods well known to the art, and/or obtained commercially as an anti-HIF-1 Beta from Nows Biologicals in Littleton, Colorado.

Optionally, Protein A plates (wet or dry) are available commercially, or the artisan can readily prepare them by standard procedures. Further, the anti-ARNT antibody is readily coated or bound onto microtiter strips or plates by other well known methods, including for instance, covalent bonding, adsorbing the anti-ARNT antibody to the plastic surface, or via a biotin-avidin system. These and further standard art procedures are described in the IMMUNOASSAY HANDBOOK, Second edition, Edited by David Wild, Nature Publishing Group, 2001, ISBN 1-56159-270-6, which is incorporated herein in its entirety by reference thereto.

Thus, a TCDD/AhR assay employing antibody capture onto microtiter strips or plates includes the following steps:
a) contacting a sample containing TCDD with AhR and nucleic acid-tracer reagent in a glass recepticle;
b) transferring the protein-nucleic acid complex to a well in a strip or plate and capturing, using standard ELISA technology techniques, the activated TCDD-Ah receptor/ARNT complex using an antibody to ARNT that is bound to the microtiter well by, for example, Protein A;
c) washing strip(s) to remove uncaptured materials;
d) detecting captured DNA response element via the nucleic acid-tracer whereby PCR reagent is added to each well of the strip(s),or plate(s) and functions to denature the complex and free the tracer during the initial heating step of the amplification. If alternative amplification techniques are employed for detection, the artisan will appreciate that, where applicable, non-thermal denaturing may be accomplished by thermal, chemical (e.g. treatment with salts, detergents, etc.) or enzymatic means.

In another embodiment of the assay method of the present invention, the capture of activated transcription factor can be facilitated by substituting a naturally occurring component of the activated transcription factor with a recombinant protein. Such as a fusion protein modified to provide an additional polypeptide sequence for ease in capturing or identifying the protein. For example, such additional polypeptide sequences may include an epitope for binding an antibody, an Fc portion of an antibody for binding protein A on a solid surface, glutathione-S-transferase, maltose binding protein, His(n), FLAG, and/or Strep-tag, to name but a few. These screenable tags are all well known in the art and are fully available to the person skilled in the art (see THE RECOMBINANT **PROTEIN HANDBOOK,** Edition AA, Amersham Pharmacia Biotech, catalog number 18-1142-75, which is incorporated herein in its entirety by reference thereto).

One such tag is a glutathione-S-transferase ("GST"), whose encoding vectors are commercially available from Amersham Pharmacia Biotech, (Piscataway, New Jersey). These vectors are employed to prepare tagged fusion moieties with a DNA binding protein of interest. The procedure for expression and purification of GST fusion proteins is widely available and sources include manufacturers such as Amersham Pharmacia Biotech and Sigma Chemical Co. For example, Amersham Pharmacia sells a GST Purification Mode as Cat. No. 27-4570-01. The procedure is scaleable from ml to liters of original culture with concomitant yields. Such tagged fusion proteins are readily isolated using standard solid phase materials such as agarose or polystyrene, that are linked covalently with glutathione or that are coated with anti-GST antibodies. As exemplified hereinbelow, vectors expressing the ARNT-GST fusion were selected and were expressed in small cultures of *Escherichia coli*. The ARNT-GST fusion protein was extracted and collected on GST-agarose (Sigma Chemical Co.).

The solid support can be any suitable substrate or surface. For ease of manipulation, this can be a commercially available reaction surface, as for example, strips of wells intended for use in PCR assays. Beads, optionally magnetic for ready manipulation, are also optionally employed.

Detection of complexed DNA (i.e. the complexed nucleic acid tracer) is accomplished by nucleic acid amplification, most simply PCR. Other nucleic acid amplification procedures such as ligase chain reaction ("LCR"), ligated activated transcription ("LAT"), rolling circular amplification technology ("RCAT) nucleic acid sequence-based amplification ("NASBA"), transcription mediated amplification ("TMA"), strand displacement amplification ("SDA") and boomerang DNA amplification (BDA) are well known in the art and may also be used.

PCR amplified DNA is readily detected by any of several well-known methods in the art, the necessary components of which are commercially available in kits. Primers, as exemplified herein for use with PCR, can also be prepared by the artisan for hybridization with the primer recognition sequences contained in the nucleic acid-tracer using PCR methods that are well known and described by Mullis, in U.S. Pat. Nos. 4,683,195 and 4,683,202, and by Wang, et al. in U.S. Pat No. 5,219,727, all of which are incorporated herein by reference thereto. Alternatively, acceptable primers may be purchased commercially from a variety of sources well known by those skilled in the art. For example, biotinylated and fluorescein-labeled primers can be purchased to allow for amplified DNA to be captured in avidin-coated wells, with peroxidase-conjugated anti-fluorescein antibodies allowing for rapid collection of quantitative data.

Those skilled in the art will understand and appreciate that when the receptor is, by way of example, AhR, the DNA responsive element within the nucleic acid-tracer is a dioxin responsive element. The nucleic acid-tracer should contain at least one DRE sequence, flanked by applicable primer recognition sequences well known to those skilled in the art. A body of work, including for example, Swanson, H.I., et al, 1995 J Biol Chem 270(44):26292-302, has shown a DRE consensus to be TNGCGTG. The B-globin sequence and a basic PCR protocol is described by Vahey, M.T., et al, PCR Primer: A Laboratory Manual, 17-21, 1995, the disclosures of which are incorporated by reference herein in their entireties.

### B. Assay Kits for Detecting Analyte

The various embodiments of the assay methods of the present invention are desirably carried out in any conventional test kit format. For example, the assay kits include supports, such as magnetic beads, capture plates or capture strips, e.g., coated with a capture material such as Protein A. The supports are optionally prepared with a capture antibody, (e.g., anti-ARNT antibodies) fixed thereto. The kit is optionally prepared to include a quantity of transcription factor, in preferably either frozen or freeze-dried form, together with other cellular components required for transcription factor activation or transformation, and nucleic acid-tracer reagent. The transcription factor and/or components necessary to the activated complex are optionally provided in the kit. The receptor (e.g., the AhR heteromer) is contacted with the analyte optionally in the presence of the nucleic acid tracer reagent and the resulting mixture is contacted with the solid support so that the resulting protein/nucleic acid complex binds to the support, preferably via the anti-ARNT antibody scheme described above. After removal of unbound material, the nucleic acid-tracer is detected and/or quantified by direct detection of captured nucleic acid-tracer by nuccleic acid amplification described herein.

### C. Assays Detecting Activated Transcription Factors

In yet another embodiment, the methods of the present invention comprise a series of steps that exploit the selective binding of an activated receptor with a nucleic acid-tracer containing a primer recognition sequence and corresponding DNA response element to form a protein-nucleic acid complex, and then determining the presence of the nucleic acid-tracer that was selectively bound, either quantitatively or qualitatively, by any art-standard nucleic acid amplification methods. In the present embodiment, the assays identify transcription factors activated *in vivo*, in order to diagnose exposure to a specific disorder, toxicant, toxin or infectious agent that is known to cause activation of the transcription factor.
These steps are summarized as:
a) extracting or obtaining activated transcription factor from cells or tissues of an animal or human to be tested;
b) allowing activated transcription factor to bind to a nucleic acid-tracer reagent comprising a nucleic acid amplification primer recognition sequence and DNA response element to form a protein-nucleic acid complex;
c) separating protein-nucleic acid complex from uncomplexed nucleic acid-tracer; and,
d) detecting and/or quantifying complexed nucleic acid-tracer by amplification and correlating to condition of interest.

The activated transcription factor is obtained from cells or tissues appropriate to the transcription factor of interest. Thus, for detection of pathogenic biomarkers, immune cells are isolated or purified from blood by standard methods (e.g., centrifugation) and fractions containing activated transcription factor are extracted from the immune cells. Preferably, the activated transcription factors are extracted uncomplexed with endogenous DNA response element,

The steps for determining the presence of the bound nucleic acid-tracer, including its separation from the unbound tracer and proteins via various capture approaches have been described supra. Where the transcription factor is AhR, IRF-1α and similar PAS family proteins, the preferred capture antibody selectively binds ARNT, the approach of which is also described supra.

Thus, an assay employing antibody capture onto microtiter strips or plates includes the following steps:
a) obtaining a sample of cells or tissue (e.g., from a cell culture, or from an animal or human patient that it is desired to test). The terms "cells" or "tissue" as used herein is intended to encompass blood and its components, unless otherwise indicated;
b) extracting a fraction from the cells or tissue containing the activated transcription factor;
c) transferring the extract believed to include the activated transcription factor into a well in a strip or plate and capture the activated complex using (ELISA technology) an antibody to ARNT that is bound to the microtiter well by Protein A;
d) washing strip(s) to remove uncaptured materials; and
e) detecting captured DNA response element via the nucleic acid-tracer whereby PCR reagent is added to each well of the strip(s) or plate(s) as described supra.

In another preferred embodiment, the capture of activated transcription factor can be facilitated by substituting a naturally occurring component of the activated transcription factor complex with a recombinant protein. Such a fusion protein can be modified to provide an additional polypeptide sequence for ease in capturing or identifying the protein as has been described in exhaustive detail supra.

The solid support can be any suitable substrate or surface as described supra along with the detection approaches for determining the protein-nucleic acid complex, also as described supra.

### D. Assay Kits for Assays for Detecting Activated Transcription Factors

The present detection method is desirably carried out in any conventional test kit format. For example, the assay kits includes supports, such as magnetic beads, capture plates or capture strips, e.g., coated with a capture material such as Protein A. The supports are optionally prepared with a capture antibody (e.g., anti-ARNT, ER, or GR antibodies) fixed thereto. The kit is prepared to include a quantity of nucleic acid-tracer specific for the transcription factor of interest, associated buffers and wash solutions.

### E. Nucleic Acid-Tracer Reagent

The nucleic acid-tracer reagent of the present invention is uniquely designed for use in detection assays incorporating nucleic acid amplification. This novel assay reagent comprises at least one nucleic acid primer recognition sequence and at least one DNA response element to which an activated transcription factor binds and can be constructed by general methods well known to those skilled in the art. In particular, the primer recognition sequence should be optimized for amplification bearing in mind that the critical parameter for optimum amplification is the correct design and position of the sequence, thereby reducing the generation of non-specific product, reducing background, and generating an amount of product close to the theoretical values of product accumulation in the exponential phase of the reaction. In exponential amplification, even a small inefficiency at each annealing step will propagate and result in a significant decrease in amplified product. (PCR Primer, A Laboratory Manual, Edited by Dieffenbach and Dvdksler, Cold Spring Harbor Laboratory Press, 1995).

Using a recognition sequence of a minimal length that ensures optimum-melting temperatures for the chosen amplification method provides the best chance for maintenance of specificity and efficiency. For PCR, primers between 18 and 24 bases tend to be very sequence-specific if the annealing temperature of the PCR is set within a few degrees of the primer Tm (defined as the dissociation temperature of the primer/template duplex). Perfect base pairing between the primer recognition sequence and primer is optimal for obtaining good results. In addition, the GC content and Tm should be well matched within primer pairs. Poorly matched primer pairs can be less efficient and specific because loss of specificity arises with a lower Tm value; the primer set with the higher Tm value has a greater chance ofmispriming under these conditions. Critical design of the primer recognition sequence will also eliminate the primer-dimer phenomenon.

There are a number of software programs designed for constructing primers that are available in the art and can be utilized in designing primer recognition sites.

As mentioned beforehand, DNA response elements are DNA sequences associated with or "compatible" with a gene or region of a gene to which a transcription factor binds and regulates transcription of the gene. The DNA response elements that have been identified to date are first presented in the literature and subsequently submitted for inclusion in sequence databases such DDBJ, EMBL, Genebank, and TRANSFAC, the contents of which are incorporated by reference herein in their entireties.

Normally, primers are designed to a preexisting nucleic acid template representing a specific sequence within a DNA or RNA molecule of interest. Under those circumstances primers are designed to obtain a balance between specificity of amplification and efficiency of amplification. In the present invention, the primer recognition sequence is designed so that the perfect primer set can be employed in the assay, allowing critical parameters in the amplification process to be optimized.

### EXAMPLES

Modifications and variations of this invention can be made without departing from its spirit and scope, as will be apparent to those skilled in the art. The specific embodiments described below are offered by way of example only, and the invention is intended to be limited only by the terms of the appended claims, along with the full scope of equivalents to which such claims are entitled.

### EXAMPLE 1

### PREPARATON OF REAGENTS

### A. Preparing Ah Receptor

The AhR-containing heteromer was obtained by standard methods from mammalian hepatocytes. These included Hepa 1c1c7 murine hepatoma cells, and rodent livers. Hepa 1c1c7 is available from the American Type Culture Collection ATCC No. CRL 2026. The rodents included C57BS/6J mice, Long-Evans rats, and Hartley guinea pigs that were obtained from Jackson Laboratories or Charles Rivers Laboratories.

Hepa 1c1c7 cells were processed as follows.

MENDG buffer was composed of 25 mM MOPS, 1 mM EDTA, 0.02% NaN3 (wt/vol), 1 mM DTT, 10% glycerol (vol/vol), 1X protease inhibitors (Sigma P2714), pH 7.5.

In brief, dishes of Hepa 1c1c7 cells were grown to confluency and washed with cold PBS. Cells were scraped in phosphate buffered saline ("PBS") and pelleted 1000 x g, 5 minutes. Cell pellets were resuspended in MENDG, 8 ml for about 50 x 150 mm plates prepared. Cells were then lysed by sonication on ice, 2 times 20 second pulses. Alternatively, cells were lysed using M-PER Mammalian Protein Extraction Reagent as per Manufacturers instructions (Pierce, Rockford, IL 61105). Lysates were ultracentrifuged at 4°C, 105,000 g, 1 hour. The supernatant, i.e., the cytosol, containing AhR heteromers was aliquoted and frozen at -70°C until used.

Rodent livers were obtained and processed as follows. Animals were sacrificed by exposure to CO₂ or cervical dislocation. The livers were removed and immediately rinsed in PBS or perfused with HEDG buffer 25 mM HEPES (N-2[2-Hydroxyethyl]piperazine-N'-[2-ethansulfonic acid] Sodium salt, 1.0 mM EDTA (Ethylenediamine-tetraacetic acid Tetrasodium salt, 1 mM DTT (DL-Dithiothreitol), 10% glycerol (vol/vol), pH 7.6.), excised and rinsed in PBS. The livers were finely minced and homogenized in ice-cold HEDG using a Teflon-glass Potter-Elvehjem tissue homogenizer. Homogenates were centrifuged at 10,000 x g for 20 minutes at 4°C. The supernatant was centrifuged at 100,000 x g for 60 minutes at 4°C. Surface lipid was removed by aspiration after each centrifugation. The cytosol containing AhR heteromers was stored at -70°C.

### B. Preparing Nucleic Acid-Tracer Specific for the Ah Receptor

Complimentary strands of the nucleic acid-tracer, designated DRE1 and DRE2 were commercially synthesized. Dioxin responsive element nucleotide sequences are known in the art and are disclosed in references provided *supra*. Primer recognition sequences were selected from primer sequences disclosed in references provided *supra*, or designed using commercially available nucleic acid software.

DRE1 (SEQ ID NO: 1)
CAACTTCATCCACGTTCACCTCGAGCTGGGGCATTGCGTGACAAGCC
GTACCTGTCCTTGGCTCTTC

DRE2 (SEQ ID NO: 2)
GAAGAGCCAAGGACAGGTACGGCTTGTCACGCAATGCCCCCAGCTCG
AGGTGAACGTGGATGAAGTTG

The underlined sequence is the dioxin responsive element and the 20 nucleotides on each end of the oligonucleotides contain the primer recognition sequences. Primers were commercially synthesized without modification (PRIA and PRIB) for gel and digoxigenin detection protocols
PRIA, GAAGAGCCAAGGACAGGTAC (SEQ ID NO: 3)
PRIB, CAACTTCATCCACGTTCACC (SEQ ID NO: 4 )

Nucleic acid-tracer molecules were prepared by annealing complementary oligonucleotide strands (e.g., DRE1 and DRE2). A 1 µM solution of complementary oligonucleotides was heated to 95°C for 1 minute, 80°C for 1 minute, 65°C for 1 minute, and then cooled to room temperature. A 10-fold dilution results in a 100 nM solution of annealed template that was aliquoted and stored at-20°C.

### C. Preparing Buffer, Protein A Strips and Capture Strips

Wash Buffer A was prepared as a composition of 10 mM Tris, 150 mM NaCl, and 0.1 % NaN₃ (wt/vol), at pH 7.6.

Protein A coated microtiter strips were prepared by coating TopYield^{tm} microtiter strips ("strips") (Nunc, Denmark) with 50 µl of Protein A, in carbonate buffer, and then incubating the strips at 37°C for 1 -1.5 hrs. Each microtiter strip consisted of 8 thin-walled wells in a format allowing the use of automated strip washers and 96-well plate compatible thermocyclers.

The Protein A coated strips were washed 3X in Wash Buffer A and 100 µl of Blocking Buffer (5% nonfat dried milk (wt/vol) in Wash Buffer A) was added to each well. The Protein A strips were stored in Blocking Buffer at 4°C, until used.

Capture Strips were then prepared using the above-described Protein A strips, as follows. Antibodies to aryl hydrocarbon receptor nuclear translocator ("ARNT") was obtained as Anti-HIF-1 Beta from Novus Biologicals, Littleton, CO) and were diluted 1/1000 in Hybrizyme Assay Buffer (commercially available from Hybrizyme, Inc., Raleigh, NC). The Protein A strips were washed 3X in Wash Buffer B (Wash Buffer A plus 0.05% Tween 20 (wt/vol)). Diluted antibody 50µl was added to each well of the Protein A strip and incubated with shaking 1-1.5 hours. The antibody coated strips were washed 3X in Wash Buffer B to remove any antibody not bound to the Protein A and used immediately.

Another antibody was also successfully employed for capture. These were rabbit anti-AhR polyclonal antibodies that were elicited to bind amino acid residues 1-402 of the murine AhR. This polyclonal antibody is described in more detail by Pollenz, RS, Sattler, CA, Poland, A Mol. Pharm., 45, 428-438 (1994), incorporated herein by reference. The rabbit anti-AhR Capture Strips was prepared as described above for the anti-ARNT antibodies.

### D. Preparing ARNT-GST Fusion Protein

A truncated cDNA, encoding from 1-474 amino acids, of murine ARNT (Reyes, H., et. al., 1992 Science 256: 1193-1195) was cloned into pGEX-4T vectors 1,2, and 3 (Cat. Nos. 27-4580-01, 27-4581-01, 27-4582-01; Amersham Pharmacia Biotech) using PCR amplified cDNA according to the procedures of Reisz-Porszasz et. al., 1994, Molecular and Cellular Biology 14(9): 6075-6086; Pongratz et. al., 1998, Molecular and Cellular Biology 18(7): 4079-4088, incorporated herein by reference in their entirety.

GST fusion protein for the following procedures was routinely prepared from 1 liter batches of bacterial culture (*E. coli BL21*) with recombinant construct. Ten ml of a saturated overnight culture, in Luria broth with ampicillin (100 µg/ml), was diluted into 1 L Luria broth with ampicillin (100 µg/ml) and incubated in a shaking water bath at 37°C for 2.5 hours. Gene expression was under *tac* promoter control and was induced by the addition of isopropyl beta thiogalactoside ("IPTG") to a final concentration of 0.1 mM, followed by an additional2.5 hours in culture. The cultured bacteria were harvested, pelleted at 7700 g, 4°C, for 10 minutes and the supernatant discarded. Bacteria were washed in PBS and re-pelleted in 2 ml tubes. Supernatants were aspirated and discarded. The resulting bacterial pellet was frozen in liquid nitrogen until processed further.

Lysis buffer (20 mM Tris, pH 8.0, 0.1 M NaCl, 10% glycerol (vol/vol) was added to the bacterial pellet and the pellet resuspended. Lysozyme was then added to 150 µg/ml of the suspended bacteria and the suspension was then incubated on ice for 30 min. DL-Dithiothreitol ("DTT") was added to a 5 mM concentration. N-laurylsarcosine (sarcosyl) was added to a 0.8% concentration (wt/vol) and cells were incubated on ice for 15 min. Bacteria were sonicated on ice for 2 times 30 seconds. Protease inhibitors (Sigma P2714) were added to final 1X concentration and lysates were ultracentrifuged at 4°C, and 40,000 g, for 45 min. Triton X-100 was added to the supernatant to 1% (vol/vol). Glutathione-agarose, 2 ml, (Sigma G4510) that had been washed in PBS was added to the extract and shaken gently for 1 hour. Agarose was washed 3 times in PBS with centrifugation at 500 g. GST-protein was eluted with batch mixing for 30 minutes in reduced glutathione, 10 mM in 50 mM Tris, pH 8.0. Purified protein was aliquoted and frozen at -20°C. A 10 µl aliquot was analyzed in SDS-PAGE, with Coomassie protein staining for visualization.

### E. Preparing Biotin Labeled ARNT

Purification of the GST fusion construct of ARNT was performed as described above. Approximately 1 ml of a 200 µg/ml solution of ARNT was dialyzed against PBS, pH 8.0 overnight at 4°C in a Slide-A-Lyzer dialysis cassette (Pierce, product numbers 66415, 66425). Using an 18 ga needle, 3, 5, or 20 µl of a 2 mg/200 µl solution of Sulfo-NHS-LC-Biotin (Pierce, product number 21430) in water was injected into the cassette. The cassette was shaken on a platform shaker for 30 min. at room temperature. The cassette was then dialyzed against 20 mM Tris, pH 8.0 overnight at 4°C.

Samples of the biotinylated ARNT exposed to either 3, 5, or 20 µl/ ml of Sulfo-NHS-LC-Biotin were analyzed by gel electrophoresis and Western blot analysis. The mobility of the ARNT preparations were slighted retarded by the addition of biotin. ARNT biotinylated at each of the Sulfo-NHS-LC-Biotin concentrations were recognized by antibodies to GST and reacted with streptavidin HRP.

### F. Preparing Neutravidin Strips

Microtiter strips (Nunc TopYield) were coated with 50µl of 5µg/ml of neutravidin (Pierce, product number 31000) in carbonate buffer and incubated at 37°C for 1hr. Strips were washed 3 times Wash Buffer A and incubated with 150 µl of Blocking Buffer (5% non fat dried milk (wt/vol) in Wash Buffer A) for 30 min at room temperature and stored at 4°C for up to two weeks. Prior to use, microtiter strips were washed 3 times Wash Buffer B.

### G. Preparing Antibody-coated Magnetic Microparticles

Anti-Hypoxia-inducible factor-1 beta (a/k/a ARNT) polyclonal antisera was purchased fromNovus Biologicals, Littleton, Co (product number NB100-110). An IgG fraction of the antisera was prepared using ImmunoPure IgG (Protein A) Purification Kit (Pierce, product number 44667) per manufacturer instructions. Approximately 1 mg IgG was prepared from 200 µl of antisera. Ser-Mag Magnetic Carboxylate-Modified Microparticles were purchased form Seradyn, Indianapolis, IN (product number 294290050250). Coupling of antibody to beads was performed following the manufacturers suggestions for preactivation covalent coupling.

During the preactivation step the following reagents were mixed in a 1.5 ml microfuge tube: 100 µl of 500 mM MES buffer, pH 6.1, 100 µl 10% solid suspension of microparticles (wt/vol), 230 µl of NHS (50 mg/ml of water), 110 µl of EDAC (10 mg/ml water) and 360 µl of water. The tube was mixed at room temperature on a mixing wheel for 30min. The microparticles were centrifuged and supernatant was discarded. The microparticles were resuspended in 1 ml of 50 mM MES buffer, pH 6.1, centrifuged and the supernatant discarded. The pellet was resuspended with 100 µl of 500 mM MES, pH 6,1, 650 µl of water and 250 µl of antibody (1 mg/ml) and rapidly mixed using a pipette. The tube was mixed at room temperature on a mixing wheel for 1 hour. The microparticles were washed 3 times with 50 mM MES, pH. 6.1. The final pellet was resuspended in either casein blocking buffer (25 mM Tris, pH 8.3, 1% casein (wt/yol), 100 mM NaCl, 0.1% NaN₃ (wt/vol)) or non-fat dried milk (NFDM) blocking buffer (25 mM Tris, pH 8.3, 5%NFDM (wt/vol), 100 mM NaCl, 0.1% NaN₃ (wt/vol).

### H. Preparation of HIF1-β Transcription Factor

### 1. Human HeLa S3 cells

Human HeLa S3 cells were maintained or treated with 125 mM CoCl₂ (Wang & Semenza, Proc. Natl. Acad. Sci. USA 1993 90:4304-4308). Cytosolic extracts were prepared from treated and untreated cells. Nuclear extracts were prepared from untreated cells as described previously (Semenza & Wang, Mol. Cell. Biol. 1992 12:5447-5454; Dignam et al., Nucleic Acids Res. 1983 11:1474-1489). HeLa S3 cells, obtained from American Type Culture Collection were grown in F 12K medium supplemented with 5% (v/v) fetal bovine serum (Life Technologies, Gaithersburg, Md.) in 150 mm tissue culture dishes.

### 2. Preparing Cytosolic Extracts

HIF-1 DNA binding activity was induced as follows. Actively dividing HeLa S3 cells were treated with 125 µM CoCl₂ for 4 hr at 37°C. Cells were washed 2X in tissue culture dishes with ice cold phosphate-buffered saline (PBS) before harvesting. Cells were pelleted at 420xg for 5 min, washed twice with PBS and resuspended in 5 packed cell volumes of buffer A (10 mM Tris-HCl (pH 7.6), 1.5 mM MgCl₂, 10 mM KCl, 1 mM DTT) supplemented with Sigma protease inhibitor cocktail (P 8340, Sigma Chemical Co., St. Louis, Mo.). After incubation on ice for 15 min, cells were pelleted at 450xg for 5 min, resuspended in 2 packed cell volumes of buffer C (0.42 M KCl, 20 mM Tris-HCl (pH 7.6), 20% glycerol, 1.5 MM MgCl₂, 1 mM DTT and 0.2 mM EDTA) supplemented with Sigma protease inhibitor cocktail. The cells were disrupted using a Branson Sonifier 450 (Branson Ultrasonics Corp., Danbury, CT) fitted with a microtip for two 20-second pulses at a 45% duty cycle. After centrifugation at 20,000xg for 5 min, the supernatant was designated as cytosolic extract. The cytosolic extracts from treated and untreated cells were aliquoted and stored at -80°C.

### 3. Preparing of Nuclear Extracts

Nuclear extracts were prepared as follows. The untreated cells as described above were pelleted at 420xg for 5 min and washed twice with ice cold phosphate-buffered saline and resuspended in 5 packed cell volumes of buffer A (10 mM Tris-HCl (pH 7.6), 1.5 mM MgCl₂, 10 mM KCl, 1 mM DTT) supplemented with Sigma protease inhibitor cocktail. After incubation on ice for 15 min, cells were pelleted at 450xg for 5 min, resuspended in 2 packed cell volumes of buffer A, and lysed by 20 strokes in a glass Dounce homogenizer with type B pestle. Nuclei were pelleted at 10,000xg for 10 min and resuspended in 2/3 of the cell pellet in buffer C (0.42 M KCl, 20 mM Tris-HCl (pH 7.6), 20% glycerol, 1.5 mM MgCl₂, 1 mM DTT and 0.2 mM EDTA) supplemented with Sigma protease inhibitor cocktail. Nuclear proteins were extracted by shaking at 4°C for 30 min. After centrifugation at 20,000xg for 5 min, the supernatant was designated as nuclear extract The nuclear extracts were aliquoted and stored at - 80°C.

### I. Preparation of Nucleic Acid-Tracer Specific for HIF-1α

Complimentary strands of the nucleic acid-tracer, designated HRE1 and HRE2 were commercially synthesized. The hypoxia responsive element (HRE) nucleotide sequences are known in the art (Semenza et. al, JBC 1996 271:32529-32537. Primer recognition sequences were selected from primer sequences disclosed in references provided *supra*, or designed using commercially available nucleic acid software.

HRE1 (SEQ ID NO: 5)
CAACTTCATCCAGTCTCACCGATCGCCCTACGTGCTGTCTCGACTCTGT
CCTTGGCTCTAC

HRE2 (SEQ ID NO:6)
GTAGAGCCAAGGACAGAGTCGAGACAGCACGTAGGGCGATCGGTGAG
ACTGGATGAAGTTG

The 20 nucleotides on each end of the oligonucleotides contain the primer recognition sequences. Primers were commercially synthesized and designated PRIHa and PRIHb.
PRIHa (SEQ ID NO: 7)
   CAACTTCATCCAGTCTCACC
PRIHb (SEQ ID NO: 8)
   GTAGAGCCAAGGACAGAGTC

The oligomers for detection of the HRE, designated HRECol and HRE Co2 were commercially synthesized without primer recognition sequences for competition assays.
HRECo1 (SEQ ID NO: 9)
   GATCGCCCTACGTGCTGTCTC
HRECo2 (SEQ ID NO:10)
   GAGACAGCACGTAGGGCGATC

Nucleic acid-tracer or DNA-competitor molecules were prepared by annealing complementary oligonucleotide strands (e.g., HRE1 and HRE2 or HRECo1, and HRECo2, respectively). A 1 µM solution of complementary oligonucleotides was heated to 95°C for 1 minute, 80°C for 1 minute, 65°C for 1 minute, and then cooled to room temperature. A 10-fold dilution results in a 100 nM solution of annealed template that was aliquoted and stored at -20°C.

### J. Preparing Estrogen Receptor Reagents

Baculovirus expressed recombinant human ER-alpha was purchased from Panvera, Madison, WI (Cat. No. P2187). Mouse monoclonal antibodies that were specific for human ER were purchased from StressGen Inc., Victoria, British Columbia, Canada (Cat. No. SRA-100) and were coated on anti-mouse IgG strips/plates (Wallac, Finland).

The nucleic acid-tracer contained a single ERE from the vitellogenin promoter, flanked by primer recognition sequences described in Example 1 B. Accordingly PRIA and PRIB were utilized as primers. All sequences are 5' to 3'

VER1 (SEQ ID NO: 11)
CAACTTCATCCACGTTCACCGTCCAAAGTCAGGTCACAGTGACCTGAT
CAAAGTTGTACCTGTCCTTGGCTCTTC

VER2 (SEQ ID NO: 12)
GAAGAGCCAAGGACAGGTACAACTTTGATCAGGTCACTGTGACCTGAC
TTTGGACGGTGAACGTGGATGAAGTTG

Competitor ERE strands
DX1 CTAGAAAGTCAGGTCACAGTGACCTG (SEQ ID NO: 13);
DX3 CAGGTCACTGTGACCTGACTTTCTAG (SEQ ID NO: 14);

### K. Preparing Glucocorticoid Receptor Reagents

Detection of activated glucocorticoid receptor (GR) was conducted by a method analogous to that of ER, *supra*. Baculovirus expressed recombinant GR was purchased from Panvera, Madison, WI (Cat. No. P2187). Rabbit polyclonal anti-GR antibodies that were specific for GR were purchased from Affinity Bioreagents, Goldin, CO (Cat. No. PA1-510A) and were coated on anti-mouse IgG strips/plates (Wallac, Finland).

The nucleic acid-tracer contains a single GRE from the human tyrosine amino transferase promoter, flanked by primer recognition sequences as described in Example 1,B. Accordingly PRIA and PRIB described above were utilized as primers. All sequences are 5' to 3'

BGGR1 (SEQ ID NO: 15);
CAACTTCATCCACGTTCACCGCTGTACAGGATGTTCTGCCGTACCTGTC
CTTGGCTCTTC

BGGR2 (SEQ ID NO: 16);
GAAGAGCCAAGGACAGGTACGGCAGAACATCCTGTACAGCGGTGAAC
GTGGATGAAGTTG

Competitor GRE strands
TGR1 GCTGTACAGGATGTTCTGCC (SEQ ID NO: 17);
TGR2 GGCAGAACATCCTGTACAGC (SEQ ID NO:18).

### EXAMPLE 2

### DETECTING DIOXINS

### A. Measuring Dioxins by Real-Time PCR

A set of calibrators consisting of 5000, 2500, 1250, 626, 313, 156, and 78 parts per trillion (ppt or pg/ml) 2,3,7,8-tetrachlorodibenzo-p-dioxin (TCDD) was prepared in methanol. In addition to TCDD a set of calibrators for AhR agonist, beta-naphthoflavone (BNF) was prepared and analyzed in the assay. Separately, 25 ul of 1:50,000 dilution of 10 uM stock nucleic acid-tracer was added per 1 ml of rodent cytosol, which was the source of AhR, and was aliquoted into glass tubes. The standards were diluted 1:20 into 50 - 100 ul of rodent cytosol, and incubated for 1- 2 hr at room temperature to 37°C. Following incubation, 40 to 100 ul of the reaction was transferred to each well of the antibody-coated Capture Strip and incubated on a shaking platform for 30 min to 1 hr at room temperature. The Capture Strips were washed 5 times with Wash Buffer B, and aspirated; optionally soaking wells for 30 sec - 5 min with wash buffer during each cycle. Taqman^{®} Universal PCR Master Mix, primers, and Taqman^{®} probe (Applied Biosystems, CA) were added, and adhesive cover applied. PCR was performed and analyzed using an ABI PRISM^{®} 7700, using default settings: 50°C, 2 min, 95°C 10 min, and 40 cycles of [95°C, 15 sec, 60°C, 60 sec]. A threshold cycle (Ct) was determined for each reaction. The Ct represents the PCR cycle at which an increase in reporter fluorescence above baseline signal can first be detected.

When AhR derived from guinea pig was employed the characteristics of the dose-response curve consisted of an EC₅₀ of 2.6 pg of TCDD in the assay, slope of-1.042 and an R-squared value of 0.9975. Similar results were obtained from a dose-response curve using AhR derived from rat consisting of an EC₅₀ of 2.8 pg of TCDD in the assay, slope of-1.208 and R-squared value of 0.9989. Representative dose-response curve data was summarized in the Table shown below.

**DOSE-RESPONSE CURVE**

| TCDD (ppt) | Ct Values |
|---|---|
| 0 | 26.29 |
| 78 | 24.52 |
| 156 | 23.81 |
| 313 | 23.16 |
| 625 | 22.23 |
| 1250 | 21.3 |
| 2500 | 20.5 |
| 5000 | 20.28 |

The pharmacology of the AhR was demonstrated by examining the reactivity of BNF in the assay as describe above. BNF is an agonist having approximately an order of magnitude less affinity for the Ah receptor than TCDD (Carver et. al., 1994 JBC 269:30109-30112.) The data from dose-response curves performed simultaneously for TCDD and BNF consisted of an EC₅₀ of 4 pM, slop -1.195, R-squared value 0.9990, and an EC₅₀ of 40 pM, slope -0.7637, R-squared value 0.9993, respectively, showing that the pharmacological properties of the AhR receptor was maintained in the assay.

Due to the advantages inherent in real-time PCR 96 samples can be analyzed in less than five hours. Additionally, real-time PCR ensures that data collection occurs in linear portion of the amplification process where assay sensitivity and precision are optimized.

### B. Measuring Dioxins by End-Point PCR

End-point PCR may also be utilized for dioxins. Calibrators containing 10, 100, 1000 fmol of 2,3,7,8-tetrachlorodibenzo-p-dioxin (TCDD) were prepared and 1 µl of each calibrator was dispensed into a glass test tube.

An extract from Hepa lclc7 cells was prepared as described by Example 1 to provide AhR for the assays. 100µl NEAT to 0.5 µl of Hepa lclc7 cell extract diluted in Assay Buffer was added to each tube, thoroughly mixed, and the tubes were capped, or covered with Parafilm^{™}. The samples were then incubated at room temperature to 37°C for 30 min to 2 hr. Salt and salmon sperm DNA was then added to the AhR reaction medium in concentrations of 50 mM to 200 mM and 5 to 100 µg/ml, respectively. The samples were then incubated for a period ranging from 0-20 minutes at room temperature. Nucleic acid-tracer (1 nM) was added and mixed gently to allow complex formation between activated AhR and nucleic acid-tracer, where TCCD was present. The reaction tubes were incubated for a time ranging from 10 min to 1 hour, at room temperature.

Capture Strips coated with anti-ARNT antibody were prepared as described in Example 1. An aliquot of AhR reaction medium (25 to 100 µl) from each glass tube was then transferred to a corresponding well of the Capture Strip. The Capture Strips were incubated for an additional 30 min to 1 hour at room temperature, with shaking. Unbound nucleic acid-tracer was rigorously removed by washing wells five times with Wash Buffer B, optionally soaking wells for 30 sec - 5 min with wash buffer during each cycle. The strips may be shaken during the soak step if desired. The strips were washed twice with water, with all liquids aspirated at the finish.

The nucleic acid-tracer remaining in the Capture Strip was PCR amplified by the following method: A PCR Master Mix was assembled with the following final concentrations: 1X PCR buffer with 1.5 mM MgCl₂, 0.2 mM dATP, dCTP, dGTP, 0.15 mM dTTP, 0.05 mM DIG-dUTP (Roche, Indianapolis, IN), 0.5 µM each primer (PRI 1 A and PRI 1B), 0.22- 0.25 µl/ reaction Taq polymerase. The Master Mix was aliquoted 30-50 µl per well, then tape applied to seal the wells. The thermocycler profile was: 94°C, 20 sec, 15 cycles of [94°C, 30 sec, 52°C, 30 sec, 72°C 30 sec], then 72°C, 30 sec and chilled to 4°C. Ten µl of PCR sample was assayed by ELISA-PCR with anti-DIG detection as follows.

The PCR sample was diluted to 30 µl with water in 0.2 ml tube. The sample was heated 95°C for 3-5 min. The immobilization (capture) probe was:
BDpro = TGTCACGCAATGCCCCCAGC-Biotin (SEQ ID NO: 19);

The probe was diluted to 10 nM in Hybridization Buffer (0.5X Assay Buffer, 0.5 M NaCl). The probe (100 µl = 1 pmol) was then added to the hot samples, mixed, and incubated at 37°C for 45 min.

The sample was transferred to a pre-washed streptavidin-coated microtiter wells in the form of strips or plates obtained from DELFIA catalog number 4009-0010 (Wallac, Finland) and shaken for 45 min. at room temperature. Strips or plates were washed three times with Wallac Wash Buffer (DELFIA wash concentrate, catalog number 13800865, Wallac, Finland). The Wallac Wash Buffer (IX) for the automated plate washer was made from 25X Wash Concentrate (Wallac), which contains Tris, NaCl, and TWEEN-20.

Anti-DIG antibody (Sigma Chemical Co, St. Louis, MO) was diluted 1/1000 in Assay Buffer and 100 µl added to each well, and strips were shaken for 30 min. Strips were then washed three times with Wallac Wash. Anti-mouse IgG that was Eu-labelled (Anti-mouse IgG-Eu, catalog number 1244-130 Wallac, Finland) was diluted 1/50 in Assay Buffer and 100 µl added to each well. The strips were shaken for 30 min and were then washed three times with Wallac Wash. Enhancement Solution (DELFIA Enhancer, catalog number C500-100, Wallac, Finland) was added (150 µl) and strips shaken for 1 min. Samples were counted on a Victor 2 multilabel counter/plate reader (catalog number 1420-012, Wallac, Finland) using time-resolved fluorescence. The amount of fluorescence detected was directly proportional to the amount of TCDD in the sample. Representative data was summarized in the dose-response curve presented in the Table below:

**DOSE RESPONSE CURVE**

| TCDD (fmol) | Counts |
|---|---|
| 0 | 5430 |
| 1 | 5770 |
| 10 | 17287 |
| 100 | 100645 |
| 1000 | 504879 |

The data generated and shown above confirms that increasing TCDD concentration results in increased production of PCR amplified DNA that incorporated the digoxigenin-modified dUTP. A standard sigmoidal dose-response curve was generated. The limit of detection for this form of the assay is between 1-10 fmol TCDD.

Similar results were obtained when the complex was immobilized by rabbit anti-AhR polyclonal antibodies, as described in Example 1.

In further testing, the addition of the AhR agonist, BNF in DMSO to the Hepa 1c1c7cell extract significantly and reproducibly increased the DNA signal over results from the control of DMSO alone.

### C. Measuring Dioxins by Employing ARNT-GST Fusion Protein

A recombinant ARNT fusion protein containing glutathione-S-transferase (ARNT/GST) was prepared so that the GST epitope could be specifically targeted with an anti-GST antibody to bind the activated Ah receptor complex. This is advantageous because the GST epitope is immunogenic and commercially prepared anti-GST microtiter plates are available from a variety of manufacturers including Pierce, Sigma Chemical Co., and Perkin Eimer Life Sciences.

The receptor was activated in the presence of ARNT/GST binding partner and test sample. Sufficient ARNT/GST was added to displace the endogenous ARNT present in the Ah receptor preparation. In a tube, 98 µl Hepalclc7 (0.8 mg) extract was incubated with 1µl GST-ARNT (50 ng) and 1µl sample (e.g. BNF or vehicle alone) at 30°C for 1 hour. Non-specific binding of the nucleic acid-tracer was reduced by preincubating the reaction mix with NaCl to 60 mM and 2 µg salmon sperm DNA for 15 min. at room temperature. Nucleic acid-tracer was added to 50 fmol and the reaction was incubated 20 min at room temperature. The complex was captured on a commercially available 96-well anti-GST plate (Perkin Elmer Life Sciences, Boston, MA). The anti-GST coated microtiter plate was washed once with Wallac Wash Buffer prior to addition of 50 µl receptor-DNA reaction. The plate was shaken 50 min at room temperature.

Unbound components were removed with a series of washes. The plate was washed 3 times in Wallac Wash Buffer, followed by addition of 0.1 ml Hybrizyme Assay Buffer to each well. After 5 minutes of shaking, the process was repeated 2 more times. After the final incubation with Assay Buffer, the buffer was aspirated and 0.1 ml water was added for 10 seconds without shaking. The water was aspirated. The commercially prepared ant-GST plates were not formatted for PCR amplification. Therefore, excess water was pre-heated to 99°C and 25 µl of the hot water was added to each well to free the bound nucleic acid-tracer for transfer to an appropriate PCR vessel (tube, strip, or plate). The samples were stored at 4°C, short-term, until PCR amplified.

The PCR was performed as described in Example 2, B. Primers, purchased lyophilized, were resuspended in TE, pH 7.5 to 100 µM. The, final reaction volume was 50µl, wherein 25 µl was from the sample described above in water. Accordingly, the Master Mix generated below was at a 2X concentration of reagents, but described as 1X for the final 50 µl volume. The final PCR consisted of 1X PCR buffer (10 mM Tris, pH 8.3, 50 mM KCl, 0.001% gelatin), 1.5 mM MgCl2, 200 µM dNTPs, 1 µM of each primer, 0.25-0.3 µl Taq polymerase, and water to bring the volume to 25 µl per reaction.

A negative control sample had 25 µl water plus 25 µl PCR Master Mix. A positive control sample had 24.5 µl water, 0.5 µl of 100 nM annealed template, and 25 µl PCR Master Mix.

The thermal cycler profile used was 95°C 30 seconds, 25-30 cycles as noted of (95°C 30 seconds, 52°C 30 seconds, 72°C 30 seconds), followed by 72°C 30 seconds. Samples were allowed to cool to room temperature before analysis, and stored at 4°C.

Samples were analyzed by agarose gel electrophoresis with visualization of ethidium-bromide stained DNA bands under UV radiation. Typically, 20 µl of each sample was analyzed in the 4% gels, yielding bright fluorescent bands from the positive controls. Gels were photographed using Polaroid 665 film, 45 second exposure, and the prints and negatives were processed. Negatives were scanned using an HPscanjet 2200c and DNA bands were quantified using ScionImage (Scion Corporation) software.

Duplicate samples were analyzed with the protocol described above with B-naphthoflavone (10 µM) or vehicle (DMSO) following 30 cycles PCR. The average value for the DMSO control samples was 363 and the average value for the samples containing BNF was 3013.

### D. Measuring Dioxins by Employing Biotin Labeled ARNT

The binding event that occurs between avidin/streptavidin-biotin is the strongest noncovalent biological interaction known. The bond formation between biotin and avidin is very rapid and stable lending itself to robust and sensitive assay systems. In this example, the Ah receptor was incubated in the presence of excess biotinyated ARNT, test sample, and nucleic acid-tracer. The activated receptor/nucleic acid-tracer complex was captured on a streptavidin coated plate.

Activation of the Ah receptor with dose was performed in a microfuge tube containing 50 µl of Ah receptor preparation, 1 µl of 200 µg/ml biotinylated ARNT, and 1 µl of 1 nM beta-naphthoflavone and incubated for 1 hr at 30°C. To minimize non- specific interactions, 1-2 µg of salmon sperm DNA was added to the activated complex, along with NaCl to a final concentration of 60 mM. After 10 min at room temperature, varying amounts of nucleic acid-tracer (50-500 fmole) was added, and the reaction allowed to incubate for an additional 10 min at room temperature. The reaction mix (40 µl) was transferred to a neutravidin-coated plate and incubated for 30 min on a Wallac plate shaker at room temperature. The wells were washed five times with Wash Buffer B, soaking wells for 3 min with wash buffer during each cycle, followed by two washes with ultrapure water.

For PCR analysis, 50 µl of PCR reagent was added and the well contents were subjected to 28 cycles of PCR amplification, using GeneAmp PCR System 2700 (Applied Biosystems, Foster City, CA). PCR was performed using 50 µl of PCR Master Mix (Promega, Madison, WI) diluted to the appropriate concentration with nuclease-free water and 1 µM of each primer was used per PCR reaction. Following the addition of PCR mix, the microtiter strip was sealed with adhesive and placed in the thermocylcer. Samples were initially heated to 95°C for 5 min to denature the Ah receptor/ARNT complex and free the nucleic acid for PCR analysis then subjected to 28 cycles of 94°C, 30 sec, 52°C, 30 sec, 72°C, 45 sec, followed by 72°C for 5 min, then cooling to 4°C.

PCR product, 20 µl, was analyzed by 4% agarose gel electrophoresis, with PCR product visualized under UV light by ethidium bromide staining. UV light-illuminated gels were photographed using a Polaroid MP 4 Land Camera and Polaroid Type 55 film. Transmission densitometry with film negatives was performed and areas under the peak calculated using arbitrary units.

As shown in the table below, biotinylated ARNT was used as an efficient capture system thereby eliminating the use of antibody. When concentrations of nucleic acid-tracer were added in excess of receptor concentration, the nucleic acid-tracer did not add appreciable to background in this assay.

**BIOTINYLATED ARNT ASSAY**

| Nucleic Acid-Tracer (fmol) | DMSO | BNF |
|---|---|---|
| 500 | 122 | 392 |
| 250 | 116 | 392 |
| 125 | 126 | 349 |
| 63 | 129 | 459 |

### E. Measuring Dioxins Employing Magnetic Microparticles

The large number of small, spherical microparticles provides a high surface area for capture antibody binding. Because these microparticles also remain suspended during reaction steps, molecular diffusion distances are short, minimizing incubation times. Magnetic microparticles allow wash steps to be carried out using small inexpensive magnets to separate unbound materials. In this example, magnetic microparticles are used instead of microtiter strips.

Activation of the Ah receptor with dose was performed in a microtiter strip containing 50 µl of Ah receptor preparation, 1µl of 200 µg/ml biotinylated ARNT, and 1µl of 1 nM beta-naphthoflavone and incubated for 1 hr at 30°C. To minimize non-specific interactions, 1-2 µg of salmon sperm DNA was added to the activated complex, along with NaCl to a final concentration of 60 mM. After 10 in room temperature, the nucleic acid-tracer (50 fmole) was added, and the reaction allowed to incubate for an additional 10 min at room temperature. Five µl of a 10% solution of microparticles coated with anti-ARNT antibody was added to each well and incubated for 30 min at room temperature on a Wallac microtiter plate shaker. The wells were washed using a using a LifeSep 96P (Dexter; Elk Grove Village, IL) magnet designed for separating magnetic particles in a microtiter plate format. The strip was placed on the magnetic separator for three minutes. The beads had migrated to the side of the well by this time and the supernatant was removed by aspiration. The beads were washed four additional times with 150 µl of Wash Buffer B. The beads were captured, supernatant removed, and resuspended in the PCR reaction mix and amplified as in Example 2, B. The amplified product was analyzed by gel electrophoresis. A significant increase in DNA was observed in BNF samples over DMSO vehicle controls.

### EXAMPLE 3

### DETECTING ACTIVATED AH RECEPTOR

To demonstrate that activated Ah receptor could be detected in cells (or tissues) that had been previously exposed to dioxins-like compounds, the following experiment was performed. Hepa lclc7 cells were grown until nearly confluent in Dulbecco's modified Eagle's medium and 8% fetal bovine serum. The cells were treated with 10 µM beta-naphthoflavone (BNF) in DMSO for 1 hour. The cells were then harvested after two washes with ice-cold PBS by centrifugation at 1000 x g. The cells were then suspended in 5 packed cell pellet volumes of 10 mM Hepes, pH 7.5 and allowed to stand for 10 min.

The suspended cells were again collected by centrifugation and resuspended in two packed cell pellet volumes of 25 mM Hepes, pH 7.5, 3 mM MgCl₂, 1 mM DTT and then lysed with 10 strokes of a Kontes all glass Dounce homogenizer (B type pestle). The resulting homogenate was checked microscopically to confirm cell lysis and then centrifuged for 10 min at 1000 x g to pellet the nuclei.

The nuclear extract was prepared as follows. The nuclei were resuspended and incubated for 30 min in 25 mM Hepes, pH 7.5, 3 mM MgCl2, 1 mM DTT containing 0.4 M KCI. The suspension was occasionally vortexed during the 30 min incubation and centrifuged in a microfuge at high speed. Glycerol was added to the supernatant to a 10% concentration. The nuclear extract was diluted to lower the salt concentration prior to testing. The diluent was Hepa lclc7 cell extract that was previously boiled in order to inactivate protein factors and cooled to room temperature. Nucleic acid-tracer (50 fmol) was added and mixed gently to allow complex formation between activated AhR and nucleic acid-tracer, where BNF was present. The reaction tubes were incubated for a time ranging from 10 min to 1 hours, at room temperature.

Capture Strips coated with anti-ARNT antibody were prepared as described in Example 1. An aliquot of AhR reaction medium (25 to 100 µl) was then transferred to a corresponding well of the Capture Strip. The Capture Strips were incubated for an additional 30 min to 1 hour at room temperature, with shaking. Unbound nucleic acid-tracer was rigorously removed by washing wells five times with Wash Buffer B, soaking wells for 2-5 min with wash buffer during each cycle. The strips were washed twice with water, with all liquids aspirated at the finish. The nucleic acid-tracer remaining in the Capture Strip was PCR amplified by the following method as described above.

When nuclear extracts from cells exposed to BNF were used in the assay, a significant and reproducible increase in PCR-amplified DNA was observed over nuclear extracts from cells treated with the vehicle, DMSO. This example provides a qualitative marker for dioxin exposure eliminating the need for extracting and testing the dioxin form the sample.

### EXAMPLE 4

### DETECTING ACTIVATED ESTROGEN RECEPTOR

In this example, antibody coated wells captured activated ER in the presence or absence of estradiol or competitor nucleic acid ERE reagent. The primer recognition sequence was eliminated from the nucleic acid sequence of the competitor ERE preventing amplification.

ER (3.5 pmol) was incubated with'or without 100 nM 17B-estradiol in Binding Buffer (25 mM Tris, pH 8.0, 50 mM KCl, 5% glycerol (vol/vol), 0.5 mM DTT, 40 µg/ml Salmon Sperm DNA) in 0.1 ml, 15 min at 4°C. Annealed competitor duplex was added to 100 fold molar excess in some samples. Nucleic acid-tracer was added (3.5 pmol) and reactions were incubated 15 min at 4°C.

Fifty µl reaction medium was incubated per microwell in previously coated anti-ER strips: These strips were made by incubation of the anti-ER antibody diluted 1/200 in Assay Buffer in anti-IgG strips for 30-45 min, shaking, followed by 3 washes in Wash Buffer B.

Following capture of the receptor-DNA complex, unbound material was washed exactly as described in Example 2, B. PCR amplification of eluted DNA was performed as stated in Example 2, B with the exception that 26 cycles of PCR were performed. Amplification products were analyzed by gel electrophoresis as described above. The baculovirus expressed recombinant human ER-alpha used in this example was not dependent upon ligand activation for DNA-binding. Both the ER treated with DMSO and Estradiol demonstrated receptor activity above background as shown in the table below.

**RECEPTOR ACTIVITY**

| Test Agent | ER Activity¹ |
|---|---|
| DMSO | 137 |
| Estradiol | 104 |
| Estradiol + comnetitor | 81 |

| | |
|---|---|
| ¹Background obtained from the negative control PCR was subtracted from data | |

The presence of competing nucleic acid reagent significantly reduced the signal demonstrating assay specificity and the ability of the assay method tao detect the state of activation of ER.

### EXAMPLE 5

### DETECTING ACTIVATED GLUCOCORTICOID RECEPTOR

In this example, antibody-coated wells captured activated GR in the presence or absence of dexamethasone or competitor GRE. Competitor GRE does not contain sequences complementary to the added primers, and therefore will not be PCR-amplified. GR (1 pmol) was incubated with or without 100 nM dexamethasone in Binding Buffer (25 mM Tris, pH 7.9, 60 mM KCl, 10% glycerol (vol/vol), 2 mM DTT, 40 µg/ml Salmon Sperm DNA) in 0.1 ml, 15 min at 4°C.

Annealed competitor duplex DNA was added to 100 fold molar excess in some samples. Nucleic acid-tracer was added (0.5 pmol) and reactions were incubated 15 min at 4°C. Fifty µl reaction was incubated per microwell in previously coated anti-GR strips. These strips were made by incubation of the anti-GR antibody diluted 1/200 in Assay Buffer in anti-IgG strips for 30-45 min, shaking, followed by 3 washes in Wash Buffer B. Following capture of the receptor-DNA complex, unbound material was washed exactly as described in Example 2, B. PCR of eluted DNA was performed as stated Example 2,B with the exception that 25 cycles of PCR were performed.

Amplification products were analyzed by gel electrophoresis as described above. Areas were corrected by subtracting the negative control value (33) and the corrected positive control value is 142. Both the GR treated with DMSO and dexamethasone demonstrated receptor activity above background as shown in the table below.

**RECEPTOR ACTIVITY**

| Test Agent | ER Activity¹ |
|---|---|
| DMSO | 82 |
| Dexamethasone | 68 |
| Dexamethasone + | competitor 44 |

| | |
|---|---|
| ¹Background obtained from the negative control PCR was subtracted from data | |

Baculovirus expressed recombinant used in this example was not dependent upon ligand activation for DNA-binding Excess nucleic acid competitor significantly reduced the signal attained by amplification. This competition demonstrates specific receptor-DNA interactions. While baculovirus expressed ER and GR are already activated, ER and GR from *in vivo* sources and other *in vitro* expression systems require ligand activation for DNA-binding to occur. Although ER and GR may be used to detect analytes specifically interacting with each receptor, respectively, in the above particular examples, they are used for demonstrating the detection of activated receptor.

### EXAMPLE 6

### DETECTING HYPOXIA INDUCIBLE FACTOR-1 ALPHA ACTIVATION

Detection of HIF-1α induction was accomplished by comparing the level of HIF-1α in untreated cells and in cells exposed to 125 mM cobalt chloride. As discussed above, cobalt, among other stimuli, is known to induce HIF-1α. The essential steps of the assay were conducted as follows.

Cytosolic extracts from control and induced Hela cells were prepared as described above. The extracts were diluted in HEDG (25mM HEPES pH 7.6, 1mM EDTA, 1mM DTT, and 10% glycerol) and 50 µl of the reaction medium added to each microfuge tube. Nucleic acid-tracer (50 fmole) was added and mixed gently to allow complex formation between HIF-1α/ARNT and tracer. In certain experiments, 100x DNA-competitor was added. The reaction tubes were incubated for 10 min at room temperature. Capture Strips coated with anti-ARNT antibody were prepared as described above. A 40 µl aliquot of the reaction medium from each microfuge tube was then transferred to a corresponding well of the Capture Strip. The Capture Strips were incubated for an additional 30 min at room temperature, with shaking. Unbound nucleic acid-tracer was rigorously removed by washing wells five times with Wash Buffer B, soaking wells for 2-5 min with wash buffer during each cycle.

The unbound nucleic acid-tracer remaining in the Capture Strip was PCR amplified by the following method.

Primers PRIHa and PRIHb (0.9 µM final) were added to 2X PCR SYBR® Green Master Mix (Applied Biosystems Cat No 4309155) and the Master Mix made 1X with Water. The DNA was eluted from the surface of the wells by adding 60 µl of Master Mix per well, applying tape'to seal the wells, and heating the strips to 95°C for 9 min in an Applied Biosystems GeneAmp 2700 thermocycler. 50 µl of master mix from each well was transferred to a PCR plate, capped and analyzed by real-time PCR using an Opticon thermocycler (MJ Research, Inc., Watham, Ma.). The thermocycler profile was: 95°C, 1 min, 40 cycles of [95°C, 15 sec, 60°C 1 min]. Melting curves were obtained to substantiate the SYBR green results.

In brief, cytosolic extracts prepared from untreated or CoCl₂ treated cells were diluted and exposed to nucleic acid-tracer. The complex was transferred to a microtiter plate coated with anti-ARNT polyclonal antibody and allowed to bind. Unbound nucleic acid-tracer was removed by washing and PCR reaction mix added. DNA amplification was monitored using real-time PCR The process takes only a few hours to complete and provides qualitative or quantitative results.

The PCR data is typically plotted as a primary growth curve. For quantitative and comparative purposes, a threshold cycle (Ct) is defined for each sample. The threshold may be an arbitrary signal above background, or a certain number of standard deviations above background. The Ct value is typically calculated near the first cycle where the PCR products are amplified at the greatest rate (no reagent is limiting) and where the amplified product is detectable above background. Detection was by carried out using a SYBR green probe. The results were summarized by in the Table 1, below.

**TABLE 1**

| | **1** | **2** | **3** | **4** |
|---|---|---|---|---|
| **Cytosol** | **Treated** | **Untreated** | **Treated** | **Untreated** |
| **DNA Competitor** | | | Plus | Plus |
| **Ct Value** | 20.47 | 23.08 | 22.52 | 22.58 |

The Ct values for treated and untreated cytosolic preparations differed by more'than 2.60 units. As a rule of thumb, a difference of one Ct unit correlates to approximately a 3 fold difference in the concentration of DNA. Addition of competitor DNA completely prevented the Unbound nucleic acid-tracer from binding the HIF-1/ARNT complex, thus demonstrating the specificity of the DNA binding.

To determine if HIF-1 could be detected in untreated cells, a nuclear extract containing more concentrated HIF-1 was prepared and the Ct values for the extract with and without the addition of competitor DNA determined. Table 2, below, shows that the addition of competitor DNA to the nuclear extract clearly inhibited DNA binding, confirming that untreated HIF-1 could be detected using the RC PCR assay.

**TABLE 2**

| **Nuclear Extract** | **1** | **2** |
|---|---|---|
| **Competitor DNA** | | Plus |
| **Ct** | 23.5 | 26.51 |

## Claims

1. An assay method for the rapid detection of analyte in a sample, said assay method comprising the steps of:
(a) contacting said sample with:
1) a transcription factor assay reagent selected from the group consisting essentially of steroid hormone superfamily receptors, PAS superfamily proteins, Stat family transcription factors, Rel orNFkB family proteins, CREB family proteins, and AP-1 family proteins capable of specific binding to said analyte and that is transformed by contact with said analyte to yield an activated transcription factor that is functional to bind a compatible DNA response element; and
2) a nucleic acid tracer reagent comprising at least one nucleic acid primer recognition sequence and at least one DNA response element to which activated transcription factor binds; said sample being contacted with said transcription factor assay reagent and said nucleic acid tracer reagent simultaneously or sequentially, under conditions promoting formation of a protein-nucleic acid complex therebetween;
(b) separating said complex from uncomplexed nucleic acid tracer reagent; and,
(c) detecting the presence of complexed nucleic acid tracer reagent by nucleic acid amplification; wherein the presence of amplified nucleic acid tracer reagent indicates the presence of analyte in the sample.

2. The assay method according to Claim 1 wherein said nucleic acid amplification is selected from the group consisting essentially of PCR, LCR, RCAT, LAT, TMA, NASBA, BDA, and SDA.

3. The assay method according to Claim 2 wherein nucleic acid amplification is real- time PCR.

4. The assay method according to Claim 1 wherein said complex is separated from uncomplexed nucleic acid tracer reagent utilizing a technique selected from the group consisting essentially of gel filtration chromatography, centrifugation filtration, hydrophobic separation, charged membrane-based separation, hydroxyapatite separation, antibody precipitation, salt precipitation, organic solvent precipitation, polyethylene glycol precipitation, and silica precipitation.

5. The assay method according to Claim 1 wherein said complex is separated from uncomplexed nucleic acid tracer reagent by contacting said complex or uncomplexed nucleic acid tracer reagent with a reagent that selectively binds to said complex or uncomplexed nucleic acid tracer reagent.

6. The assay method according to Claim 5 wherein said complex is separated from uncomplexed nucleic acid tracer reagent by contacting said complex with an antibody that selectively binds to an epitope of said complex.

7. The assay method according to Claim 5 wherein said reagent is immobilized on a solid support.

8. The assay method according to Claim 7 wherein the support is selected from the group consisting essentially of test tubes, microtiter wells, microtiter strips, microtiter plates, beads, microparticles, magnetic particles and combinations thereof.

9. The assay method according to Claim 5 wherein said complex is biotinylated and is separated from uncomplexed nucleic acid tracer reagent by contacting said complex with avidin bound to a solid phase.

10. Use of a nucleic acid tracer reagent in an assay for the detection of an analyte in a sample, said nucleic acid tracer reagent comprising at least one nucleic acid amplification primer recognition sequence and at least one DNA response element for binding of a transcription factor, wherein said transcription factor is capable of specific binding to the analyte and wherein the transcription factor is transformed by binding of the analyte to yield an activated transcription factor that is functional to bind to said DNA response element.

11. Use according to claim 10, wherein said assay comprises a nucleic acid amplification reaction using said primer recognition sequence as annealing site for a primer in a nucleic acid amplification reaction for amplification of said nucleic acid-tracer reagent, wherein the presence of amplified nucleic acid tracer reagent indicates the presence of analyte in the sample.

12. Use according to claim 10, wherein said nucleic acid amplification primer recognition sequence is optimized for amplification using one the group of techniques selected from PCR, LCR, RCAT, LAT, TMA, NASBA, BDA, and SDA.

13. Use according to claim 10, wherein said nucleic acid amplification primer recognition sequence is optimized for amplification by PCR.

14. Use according to claim 10, wherein said DNA response element is selected from the group consisting essentially of dioxin-response elements, estrogen- response elements, and glucocorticoid-response elements, and HIF-1a response element.

## Patentansprüche

1. Testverfahren zur schnellen Bestimmung einer zu analysierenden Substanz in einer Probe, das folgende Schritte umfasst:
(a) Inkontaktbringen der Probe mit
1) einem Transkriptionsfaktor-Testreagens, das aus der Gruppe ausgewählt ist, die im Wesentlichen besteht aus Rezeptoren für die Steroidhormon-Oberfamilie, Proteinen der PAS-Oberfamilie, Transkriptionsfaktoren der Stat-Familie, Proteinen der Rel-Familie oder der NFkB-Familie, Proteinen der CREB-Familie und Proteinen der AP-1-Familie, die befähigt sind, eine spezifische Bindung mit der zu analysierenden Substanz einzugehen, und das durch Kontakt mit der zu analysierenden Substanz in einen aktivierten Transkriptionsfaktor umgewandelt wird, der die Funktionalität besitzt, eine Bindung mit einem kompatiblen DNA-Reaktionselement einzugehen, und
2) einem Nucleinsäure-Tracerreagens, das mindestens eine Nucleinsäureprimer-Erkennungssequenz und mindestens ein DNA-Reaktionselement umfasst, an das der aktivierte Transkriptionsfaktor bindet, wobei die Probe mit dem Transkriptionsfaktor-Testreagens und dem Nucleinsäure-Tracerreagens gleichzeitig oder nacheinander unter Bedingungen in Kontakt gebracht wird, welche die Bildung eines Protein-Nucleinsäure-Komplexes zwischen ihnen fördern;
(b) Abtrennen dieses Komplexes von nicht komplexgebundenem Nucleinsäure-Tracerreagens
und
(c) Erfassen des Vorliegens von komplexgebundenem Nucleinsäure-Tracerreagens durch Nucleinsäure-Amplifizierung, wobei das Vorliegen von amplifiziertem Nucleinsäure-Tracerreagens das Vorliegen der zu analysierenden Substanz in der Probe anzeigt.

2. Testverfahren nach Anspruch 1, bei dem die Nucleinsäure-Amplifizierung aus der Gruppe von Verfahren ausgewählt wird, die im Wesentlichen besteht aus PCR, LCR, RCAT, LAT, TMA, NASBA, BDA und SDA.

3. Testverfahren nach Anspruch 2, bei dem die Nucleinsäure-Amplifizierung durch Echtzeit-PCR erfolgt.

4. Testverfahren nach Anspruch 1, bei dem der Komplex von nicht komplexgebundenem Nucleinsäure-Tracerreagens durch Anwendung eines Verfahrens abgetrennt wird, das aus der Gruppe ausgewählt ist, die im Wesentlichen besteht aus Gelfiltrationschromatographie, Zentrifugieren, Filtration, hydrophober Trennung, Trennung auf der Basis einer geladenen Membran, Hydroxyapatit-Trennung, Antikörperfällung, Salzfällung, Fällung mit einem organischen Lösungsmittel, Fällung mit Polyethylenglycol und Fällung mit Kieselsäure.

5. Verfahren nach Anspruch 1, bei dem der Komplex **dadurch** von nicht komplexgebundenem Nucleinsäure-Tracerreagens abgetrennt wird, dass der Komplex oder das nicht komplexgebundene Nucleinsäure-Tracerreagens mit einem Reagens in Kontakt gebracht wird, das an dem Komplex oder dem nicht komplexgebundenen Nucleinsäure-Tracerreagens bindet.

6. Testverfahren nach Anspruch 5, bei dem der Komplex **dadurch** von nicht komplexgebundenem Nucleinsäure-Tracerreagens abgetrennt wird, dass der Komplex mit einem Antikörper in Kontakt gebracht wird, der selektiv an einem Epitop des Komplexes bindet.

7. Testverfahren nach Anspruch 5, bei dem das Reagens auf einem festen Träger immobilisiert ist.

8. Testverfahren nach Anspruch 7, bei dem der Träger aus der Gruppe ausgewählt ist, die im Wesentlichen besteht aus Reagenzröhrchen, Mikrotitervertiefungen, Mikrotiterstreifen, Mikrotiterplatten, Kügelchen, Mikropartikeln und magnetischen Partikeln sowie Kombinationen davon.

9. Testverfahren nach Anspruch 5, bei dem der Komplex biotinyliert und **dadurch** von nicht komplexgebundenem Nucleinsäure-Tracerreagens abgetrennt wird, dass der Komplex mit an einer festen Phase gebundenem Avidin in Kontakt gebracht wird.

10. Verwendung eines Nucleinsäure-Tracerreagens in einem Assay zur Erfassung einer zu analysierenden Substanz in einer Probe, wobei das Nucleinsäure-Tracerreagens mindestens eine Erkennungssequenz für einen Nucleinsäureamplifizierungs-Primer und mindestens ein DNA-Reaktionselement zur Bindung eines Transkriptionsfaktors umfasst, wobei der Transkriptionsfaktor befähigt ist, eine spezifische Bindung mit der zu analysierenden Substanz einzugehen, und wobei der Substanz in einen aktivierten Transkriptionsfaktor umgewandelt wird, der die Funktionalität besitzt, eine Bindung mit dem DNA-Reaktionselement einzugehen.

11. Verwendung nach Anspruch 10, wobei das Assay eine Nucleinsäure-Amplifizierungsreaktion umfasst, bei der die Primer-Erkennungssequenz als Reassoziierungsstelle für einen Primer in einer Nucleinsäure-Amplifizierungsreaktion zur Amplifizierung des Nucleinsäure-Tracerreagens verwendet wird, wobei das Vorliegen von amplifiziertem Nucleinsäure-Tracerreagens das Vorliegen der zu analysierenden Substanz in der Probe anzeigt.

12. Verwendung nach Anspruch 10, wobei die Erkennungssequenz für den Nucleinsäureamplifizierungs-Primer für eine Amplifizierung optimiert ist, bei der ein Verfahren aus der Gruppe von Verfahren angewandet wird, das ausgewählt ist unter PCR, LCR, RCAT, LAT, TMA, NASBA, BDA und SDA.

13. Verwendung nach Anspruch 10, bei dem die Erkennungssequenz für den Nucleinsäureamplifizierungs-Primer für die Amplifizierung durch PCR optimiert ist.

14. Verwendung nach Anspruch 10, wobei das DNA-Reaktionselement aus der Gruppe ausgewählt ist, die im Wesentlichen besteht aus Dioxin-Reaktionselementen, Östrogen-Reaktionselementen und Glucocorticoid-Reaktionselementen sowie dem HIF-1 a-Reaktionselement.

## Revendications

1. Procédé de dosage permettant de détecter rapidement un analyte dans un échantillon, ledit procédé de dosage comprenant les étapes consistant à :
(a) mettre ledit échantillon en contact avec :
1) un réactif de dosage de facteur de transcription, choisi dans le groupe essentiellement constitué par des récepteurs de la superfamille des hormones stéroïdes, des protéines de la superfamille PAS, des facteurs de transcription de la famille Stat, des protéines des familles Rel ou NFkB, des protéines de la famille CREB et des protéines de la famille AP-1, capable de se lier spécifiquement audit analyte et transformé par le contact avec ledit analyte pour donner un facteur de transcription activé qui est fonctionnel pour la liaison à un élément de réponse d'ADN compatible ; et
2) un réactif traceur d'acide nucléique comprenant au moins une séquence de reconnaissance d'amorce d'acide nucléique et au moins un élément de réponse d'ADN auquel le facteur de transcription activé se lie ; ledit échantillon étant mis en contact avec ledit réactif de dosage de facteur de transcription et ledit réactif traceur d'acide nucléique simultanément ou séquentiellement, dans des conditions qui favorisent la formation d'un complexe protéine/acide nucléique entre eux ;
(b) séparer ledit complexe du réactif traceur d'acide nucléique non complexé ; et
(c) détecter la présence de réactif traceur d'acide nucléique complexé par amplification d'acide nucléique ; dans lequel la présence de réactif traceur d'acide nucléique amplifié indique la présence de l'analyte dans l'échantillon.

2. Procédé de dosage selon la revendication 1, dans lequel ladite amplification d'acide nucléique est choisie dans le groupe essentiellement constitué par les techniques PCR, LCR, RCAT, LAT, TMA, NASBA, BDA et SDA.

3. Procédé de dosage selon la revendication 2, dans lequel l'amplification d'acide nucléique est la PCR en temps réel.

4. Procédé de dosage selon la revendication 1, dans lequel ledit complexe est séparé du réactif traceur d'acide nucléique non complexé en utilisant une technique choisie dans le groupe comprenant essentiellement la chromatographie par filtration sur gel, la filtration par centrifugation, la séparation hydrophobe, la séparation à base de membrane chargée, la séparation sur hydroxyapatite, la précipitation d'anticorps, la précipitation de sel, la précipitation par solvant organique, la précipitation par le polyéthylène glycol et la précipitation de silice.

5. Procédé de dosage selon la revendication 1, dans lequel ledit complexe est séparé dudit réactif traceur d'acide nucléique non complexé en mettant ledit complexe ou le réactif traceur d'acide nucléique non complexé en contact avec un réactif qui se lie sélectivement audit complexe ou au réactif traceur d'acide nucléique non complexé.

6. Procédé de dosage selon la revendication 5, dans lequel ledit complexe est séparé du réactif traceur d'acide nucléique non complexé en mettant ledit complexe en contact avec un anticorps qui se lie sélectivement à un épitope dudit complexe.

7. Procédé de dosage selon la revendication 5, dans lequel ledit réactif est immobilisé sur un support solide.

8. Procédé de dosage selon la revendication 7, dans lequel le support est choisi dans le groupe essentiellement constitué par des tubes à essais, des puits de microtitration, des bandes de microtitration, des plaques de microtitration, des billes, des microparticules, des particules magnétiques et des combinaisons de ces éléments.

9. Procédé de dosage selon la revendication 5 dans lequel ledit complexe est biotinylé et séparé du réactif traceur d'acide nucléique non complexé en mettant ledit complexe en contact avec de l'avidine liée à une phase solide.

10. Utilisation d'un réactif traceur d'acide nucléique dans un dosage servant à détecter un analyte dans un échantillon, ledit réactif traceur d'acide nucléique comprenant au moins une séquence de reconnaissance d'amorce d'amplification d'acide nucléique et au moins un élément de réponse d'ADN pour la liaison à un facteur de transcription, dans laquelle ledit facteur de transcription est capable de se lier spécifiquement à l'analyte et dans laquelle le facteur de transcription est transformé par la liaison à l'analyte pour donner un facteur de transcription activé qui est fonctionnel pour la liaison audit élément de réponse d'ADN.

11. Utilisation selon la revendication 10, dans laquelle ledit dosage comprend une réaction d'amplification d'acide nucléique utilisant ladite séquence de reconnaissance d'amorce comme site de circularisation d'amorce dans une réaction d'amplification d'acide nucléique servant à amplifier ledit réactif traceur d'acide nucléique, où la présence de réactif traceur d'acide nucléique amplifié indique la présence d'analyte dans l'échantillon.

12. Utilisation selon la revendication 10, dans laquelle ladite séquence de reconnaissance d'amorce d'amplification d'acide nucléique est optimisée pour l'amplification en utilisant une technique choisie dans le groupe de techniques PCR, LCR, RCAT, LAT, TMA, NASBA, BDA et SDA.

13. Utilisation selon la revendication 10, dans laquelle ladite séquence de reconnaissance d'amorce d'amplification d'acide nucléique est optimisée pour l'amplification par PCR.

14. Utilisation selon la revendication 10, dans laquelle ledit élément de réponse d'ADN est choisi dans le groupe essentiellement constitué par des éléments de réponse à la dioxine, des éléments de réponse aux oestrogènes, des éléments de réponse aux glucocorticoïdes et l'élément de réponse HIF-la.
